(19) Europäisches Patentamt / European Patent Office / Office européen des brevets

(11) EP 2 899 275 B1

(12) **EUROPEAN PATENT SPECIFICATION**

(45) Date of publication and mention of the grant of the patent:
**27.06.2018 Bulletin 2018/26**

(21) Application number: **13839246.9**

(22) Date of filing: **19.09.2013**

(51) Int Cl.:
***C12N 15/09*** *(2006.01)* ***C12Q 1/04*** *(2006.01)*
***C12Q 1/68*** *(2018.01)*

(86) International application number:
**PCT/JP2013/075337**

(87) International publication number:
**WO 2014/046200 (27.03.2014 Gazette 2014/13)**

(54) **METHOD FOR OBTAINING INFORMATION ABOUT ENDOMETRIAL CANCER, AND MARKER AND KIT FOR OBTAINING INFORMATION ABOUT ENDOMETRIAL CANCER**

VERFAHREN ZUR GEWINNUNG VON INFORMATIONEN ÜBER ENDOMETRIUMKARZINOM SOWIE MARKER UND KIT ZUR GEWINNUNG VON INFORMATIONEN ÜBER ENDOMETRIUMKARZINOM

MÉTHODE PERMETTANT D'OBTENIR DES INFORMATIONS SUR UN CANCER DE L'ENDOMÈTRE, MARQUEUR ET KIT ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.09.2012 JP 2012205769**

(43) Date of publication of application:
**29.07.2015 Bulletin 2015/31**

(73) Proprietors:
- **SYSMEX CORPORATION**
  **Chuo-ku**
  **Kobe-shi**
  **Hyogo 651-0073 (JP)**
- **The University of Tokyo**
  **Bunkyo-ku,**
  **Tokyo 113-8654 (JP)**

(72) Inventors:
- **SAKAI, Ayako**
  **Kobe-shi**
  **Hyogo 651-0073 (JP)**
- **NAGAE, Genta**
  **Tokyo 113-8654 (JP)**
- **KAJITA, Masahiro**
  **Kobe-shi**
  **Hyogo 651-0073 (JP)**
- **ABURATANI, Hiroyuki**
  **Tokyo 113-8654 (JP)**

(74) Representative: **Paemen, Liesbet R.J. et al**
**De Clercq & Partners**
**Edgard Gevaertdreef 10 a**
**9830 Sint-Martens-Latem (BE)**

(56) References cited:
**JP-A- 2007 531 512** **JP-A- 2011 160 711**

- **DIANA L. KOLBE ET AL: "Differential Analysis of Ovarian and Endometrial Cancers Identifies a Methylator Phenotype", PLOS ONE, vol. 7, no. 3, 5 March 2012 (2012-03-05), page e32941, XP055267312, DOI: 10.1371/journal.pone.0032941**
- **D. FURLAN: "The High Frequency of De novo Promoter Methylation in Synchronous Primary Endometrial and Ovarian Carcinomas", CLINICAL CANCER RESEARCH, vol. 12, no. 11, 1 June 2006 (2006-06-01), pages 3329-3336, XP055267301, US ISSN: 1078-0432, DOI: 10.1158/1078-0432.CCR-05-2679**
- **JOJI IMURA ET AL.: 'Shikyutaibu no Tadankai Hatsugan Katei ni Okeru Methyl-ka no Chikuseki' NIPPON BYORI GAKKAI KAISHI vol. 96, no. 1, 2007, page 175, XP008178678**
- **VELASCO,A. ET AL.: 'Promoter hypermethylation and expression of sprouty 2 in endometrial carcinoma.' HUM.PATHOL. vol. 42, 2011, pages 185 - 193, XP027592331**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- LEVINE,D. ET AL.: 'The Cancer Genome Atlas (TCGA) project on endometrial carcinoma: Initial data and preliminary genomic analysis.' GYNECOL. ONCOL. vol. 125, no. 3, June 2012, page 772, XP028920889

## Description

### TECHNICAL FIELD

The present invention relates to a method for obtaining information on uterine body cancer in a subject. The present invention also relates to use of a kit in the method.

### BACKGROUND ART

The uterus contains the cervix corresponding to the entrance and the corpus that is located behind the cervix. Uterine body cancer is cancer of the corpus of the uterus, develops in the endometrium and thus is also referred to as endometrial cancer. Uterine body cancer is generally examined by pathological examination (cytological examination and biopsy) in which endometrial cells and tissues are collected and observed by microscopy. However, the pathological examination requires insertion of a special instrument for cell or tissue collection into the corpus of the uterus, which may be accompanied by pain and bleeding and thus put a great burden on subjects.

Examination for uterine body cancer includes measurement of tumor markers in blood. The tumor markers used include CA125, CA19-9 and the like. However, the examination of the tumor markers has insufficient cancer detection sensitivity. The examination has also been utilized for examinations of types of cancer different from uterine body cancer, and thus has insufficient sensitivity and specificity as the specific screening examination for uterine body cancer.

Meanwhile, new diagnosis methods for cancer have been recently studied which are based on genetic information. The methods include, for example, ones based on information on methylation of DNAs. The methods use markers which are CpG sites (5'-(CG)-3') in base sequences of certain genes. Based on the analysis results of the methylation status of the markers, information such as presence or absence of a cancer cell is obtained, which information may be used as an index for diagnosis of cancer.

Methods for determining cancer utilizing methylation analyses of DNA have been studied and developed for uterine body cancer. For example, the publication by Esteller M. et al discloses that MLH1 gene is not methylated in normal endometrial tissue while it is highly methylated in tissues of endometrial cancer (see Non-Patent Literature 1). The publication by Furlan D. et al. discloses that three genes, MLH1, CDKN2A and MGMT are highly methylated in tumor tissues of the endometrium and ovary (see Non-Patent Literature 2). Kolbe et al. (see Non-Patent Literature 3) discloses the examination of the methylation state of 27578 CpG sites representing 14495 genes in the human genome, using the HumanMethylation27 Illumina BeadChip and DNA derived from ovarian and endometrial tumor and normal samples. Different methylation markers for different gynaecological cancers including endometrium or uterine body cancer are reported.

Citation List

Non-Patent Literature

Non-Patent Literature 1: Esteller M. et al., Am. J. Pathol. vol. 155, p. 1767-1772 (1996)
Non-Patent Literature 2: Furlan D. et al., Clin. Cancer Res. vol. 12, p. 3329-3336 (2006)
Non-Patent Literature 3: Kolbe et al., Plos One, 7(3), e32941 (2012)

### SUMMARY OF THE INVENTION

Although the genes indicative of abnormal methylation in uterine body cancer have been reported as described above, these genes are methylated in some extent in a type of cancer different from uterine body cancer. When methylation analysis is carried out with a marker that is methylated in several types of cancer including uterine body cancer, information deduced from the analysis result may include not only information on uterine body cancer but also information on other types of cancer. In this case, even when an analysis result provides information indicating that a sample contains a cancer cell for example, it is difficult to determine whether the cancer cell is derived from uterine body cancer or from other types of cancer.

As such upon obtaining information on uterine body cancer by methylation analysis of a marker, the specificity of the marker to uterine body cancer is very important. Thus there is a need for a novel marker specific to uterine body cancer, which allows specific detection of a cancer cell derived from uterine body cancer.

Accordingly an object of the present invention is to provide a method for obtaining information on the presence or absence of a cancer cell derived from uterine body cancer in a biological sample collected from a subject by identifying such a novel marker and analyzing methylation status of the marker. Another object of the present invention is the use of a kit for the method.

The inventors of the present invention have identified novel markers which are genetic regions specifically methylated in DNAs obtained from cancerous tissues of uterine body cancer. The inventors of the present invention have found that cancer cells derived from uterine body cancer can be clearly discriminated from other cells (cells of normal tissues, cells of non-cancerous tissues and cancer cells derived from a type of cancer other than uterine body cancer) based on the result obtained by analyzing methylation status of the markers, thereby completing the present invention.

Thus the present invention provides a method for obtaining information on the presence or absence of a cancer cell derived from uterine body cancer in a biological sample collected from a subject comprising the steps of:

preparing a DNA sample from the biological sample collected from the subject;
analyzing methylation status of a CpG site in a promoter region of at least one gene selected from F13A1 (Coagulation factor XIII, A1 polypeptide) and CHCHD5 (Coiled-coil-helix-coiled-coil-helix domain containing 5) in the DNA sample obtained from the preparation step; and
obtaining information on the presence or absence of a cancer cell derived from uterine body cancer in thebiological sample collected from the subject based on an analysis result obtained from the analyzing step.

The present invention also provides use of a kit for obtaining information on the presence or absence of a cancer cell derived from uterine body cancer in a biological sample collected from a subject, said kit comprising a primer set for analysis of methylation status of at least one CpG site selected from CpG sites located in promoter regions of F13A1 and CHCHD5 genes.

## BRIEF DESCRIPTION OF THE DRAWINGS

Fig. 1A is a graph showing the methylation positive rate of the promoter region of F13A1 gene calculated from methylation data of cancerous tissues of uterine body cancer and normal uterine tissue;
Fig. 1B is a graph showing the methylation positive rate of the promoter region of CHCHD5 gene calculated from methylation data of cancerous tissues of uterine body cancer and normal uterine tissue;
Fig. 2A is a graph showing the methylation positive rate of the promoter region of F13A1 gene calculated from methylation data of various clinical specimens;
Fig. 2B is a graph showing the methylation positive rate of the promoter region of CHCHD5 gene calculated from methylation data of various clinical specimens;
Fig. 3A is a graph showing the methylation positive rate of the promoter region of a known marker gene MLH1 calculated from methylation data of various clinical specimens;
Fig. 3B is a graph showing the methylation positive rate of the promoter region of a known marker gene CDKN2A calculated from methylation data of various clinical specimens;
Fig. 4A is a graph showing the methylation score of the promoter region of F13A1 gene in DNAs extracted from normal uterine tissues and cancerous tissues of uterine body cancer;
Fig. 4B is a graph showing the methylation score of the promoter region of CHCHD5 gene in DNAs extracted from normal uterine tissues and cancerous tissues of uterine body cancer;
Fig. 5 is an image showing the results of amplification by methylation specific PCR (MSP) of DNAs extracted from normal uterine tissue and cancerous tissues of uterine body cancer using the respective primer sets for F13A1 and CHCHD5;
Fig. 6A is a bar graph showing the band intensity of amplification products obtained by MSP of DNA extracted from peripheral blood of uterine body cancer patients and healthy subjects with a primer set for F13A1 (SEQ ID NOs: 9 and 10);
Fig. 6B is a bar graph showing the band intensity of amplification products obtained by MSP of DNA extracted from peripheral blood of uterine body cancer patients and healthy subjects with a primer set for accuracy control (SEQ ID NOs: 15 and 16);
Fig. 7 is a schematic view of an example of a determination device for providing information on uterine body cancer in a subject;
Fig. 8 is a block diagram showing the functionality configuration of the determination device of Fig. 7;
Fig. 9 is a block diagram showing the hardware configuration of the determination device shown in Fig. 7; and
Fig. 10 is a flow chart of determination for providing information on uterine body cancer in a subject using the determination device in Fig. 7.

## MODES FOR CARRYING OUT THE INVENTION

In the method for obtaining information on uterine body cancer described herein (hereinafter also merely referred to as "method"), a DNA sample is first prepared from a biological sample collected from a subject.

The biological sample is not particularly limited as far as it is a biological sample containing DNA of a subject and is preferably a sample containing a genomic DNA such as a clinical specimen. The clinical specimen may include, for example, body fluid, urine, tissues obtained by operations or biopsies and the like. The body fluid may include blood, serum, plasma, lymph fluid, ascetic fluid, bone marrow aspirate, nipple discharge and the like. The biological sample may also be a culture medium obtained by culturing cells or tissues collected from a subject.

The DNA sample can be prepared by extracting DNA from the biological sample. The method for extracting DNA from biological samples is well known in the art. Extraction of DNA can be carried out, for example, by mixing the biological sample with a treatment solution containing a surfactant for solubilization of cells or tissues (e.g. sodium cholate, sodium dodecyl sulfate etc.), and subjecting the resulting mixture to physical procedure (stirring, homogenization, ultrasonication etc.) to release DNA contained in the biological sample into the mixture. In this case, it is preferable to centrifuge the mixture to precipitate cell debris and use the supernatant containing the released DNA to the next step of analyzing. The obtained supernatant may be purified according to well-known methods. DNA can also be extracted and purified from a biological sample by using commercially available kits.

The preparation step preferably further comprises the step of fragmenting the extracted DNA. By fragmenting DNA to have appropriate length, methylated DNA immunoprecipitation (MeDIP) and non-methylated cytosine conversion as described hereinbelow can be effectively carried out.

Fragmentation of DNA may be carried out by ultrasonication, alkaline treatment, restriction enzyme treatment and the like. When DNA is fragmented by alkaline treatment, a sodium hydroxide solution may be added to a DNA solution to the final concentration of 0.1N to 1.0N and the mixture may be incubated at 10°C to 40°C for 5 to 15 minutes to fragment the DNA. When the restriction enzyme treatment is used for DNA fragmentation, the restriction enzyme may appropriately be selected based on the base sequence of DNA, which may be *M*seI or *Bam*HI, for example.

According to the present method, methylation status of a CpG site in a promoter region of at least one gene selected from F13A1 and CHCHD5 in DNA obtained from the preparation step is analyzed.

As used herein, the term "CpG site" means a site of a sequence in which cytosine (C) and guanine (G) are adjacent in this order from 5' to 3'. The letter "p" in "CpG" represents a phosphodiester bond between cytosine and guanine.

As used herein, to "analyze methylation status" means to analyze presence or absence of methylation of a CpG site located in a promoter region of at least one gene selected from F13A1 and CHCHD5 or analyze methylation frequency in the promoter region.

The base sequences *per se* of the promoter regions of F13A1 and CHCHD5 genes are well known in the art. The base sequences can be obtained from well-known databases such as the one provided by the National Center for Biotechnology Information (NCBI) (http://www.ncbi.nlm.nih.gov/). The ID numbers of the above genes are shown in Table 1. The base sequences of the promoter regions of the genes (sequences of negative strands) are represented by SEQ ID NOs: 1 and 2, respectively.

Table 1

| **Gene symbol** | Unigene ID | Entrez Gene ID | Transcript ID | **SEQ ID NO:** |
|---|---|---|---|---|
| *F1341* | Hs. 335513 | 2162 | NM_000129,3 | 1 |
| *CHCHD5* | Hs. 375707 | 84269 | NM_032309.2 | 2 |

In embodiments of the present method, the analyzing step may be the step of analyzing presence or absence of methylation of at least one CpG site among CpG sites located in a promoter region of at least one gene selected from F13A1 and CHCHD5. The term "presence or absence of methylation" means whether or not cytosine in a CpG site located in the promoter region is methylated. One CpG site may be analyzed; however, more than one CpG site is preferably analyzed for presence or absence of methylation. More than one CpG site may be selected from a promoter region of one gene or from each of promoter regions of more than one gene.

In other embodiments of the present method, the analyzing step may be the step of analyzing methylation frequency in a promoter region of at least one gene selected from F13A1 and CHCHD5. The term "methylation frequency" means the ratio of the number of methylated CpG site(s) relative to the number of CpG site(s) located in the promoter region. The target for analysis may be the whole promoter region or a part thereof including at least one CpG site. The target for analysis may contain only one CpG site; however it is preferable that the target for analysis contains more than one CpG site. The target for analysis may be selected from any one promoter region among the above genes or from promoter regions of more than one gene.

The positions and numbers of CpG sites located in the promoter regions of F13A1 and CHCHD5 genes are already known. Thus the number of methylated CpG site(s) itself in the promoter regions can also be used as the methylation frequency.

The methylation frequency may be "methylation score" obtained by analyzing DNA for methylation status of CpG sites with mass spectrometry such as MassARRAY® as described hereinbelow. MassARRAY® allows calculation of methylation score based on the ratio between the area of a peak derived from a methylated DNA fragment and the area of a peak derived from a non-methylated DNA fragment obtained after measurement of the DNA fragments.

The target for analysis may be any CpG site(s) (or certain region(s) including the CpG site(s)) in the promoter regions of F13A1 and CHCHD5 genes without particular limitation and may be appropriately selected by a person skilled in the art. The positions and numbers of CpG sites located in the promoter regions of the genes are already known. Thus the target CpG site or region may be selected by routine experiments according to the well known analysis methods described hereinbelow.

Various methods are well-known in the art as to the method for analyzing methylation status. According to the present method, it is not specifically limited as to which analysis method is used; however, the analysis method preferably comprises differentiating methylated DNA from non-methylated DNA, amplifying DNA and detecting methylated DNA and/or non-methylated DNA.

The step of differentiating methylated DNA from non-methylated DNA may include the step of carrying out methylation sensitive restriction enzyme treatment, the MeDIP method, non-methylated cytosine converting treatment and the like.

The step of amplifying DNA may include the step of carrying out PCR, quantitative PCR, IVT (*in vitro* transcription) amplification, SPIA™ amplification and the like methods.

The step of detecting methylated DNA and/or non-methylated DNA may include the step of carrying out electrophoresis, sequence analysis, microarray analysis, mass spectrometry, Southern hybridization and the like.

The MeDIP method is a method in which methylated DNA in a biological sample is concentrated by immunoprecipitation using an anti-methylated cytosine antibody or an anti-methylated cytidine antibody, or an antibody which specifically recognizes a methylated DNA-binding protein. In embodiments of the present method, the analyzing step may be the step of concentrating methylated DNA in DNA obtained in the extracting step by the MeDIP method and analyzing methylation status of the concentrated methylated DNA. The methylated DNA concentrated by the MeDIP method may be amplified by e.g. IVT amplification and methylation status of the obtained amplified product may be analyzed by using a microarray. These analysis procedures are referred to as the MeDIP on chip method.

The non-methylated cytosine converting treatment is the one in which DNA extracted from a biological sample is subjected to reaction with a non-methylated cytosine conversion agent so as to convert non-methylated cytosine(s) in the DNA to a different base (uracil, thymine, adenine or guanine). In this context, the non-methylated cytosine conversion agent is an agent which can react with DNA and convert a non-methylated cytosine in the DNA to a different base (uracil, thymine, adenine or guanine). The non-methylated cytosine conversion agent suitably used may be, for example, bisulfite such as sodium, potassium, calcium or magnesium bisulfite.

In the treatment using bisulfite, non-methylated cytosine(s) in DNA is converted to uracil due to deamination reaction, while a methylated cytosine does not undergo such a base conversion.

Thus, the difference in methylation status of a CpG site in DNA is converted to the difference in base sequence (C and U) by the non-methylated cytosine converting treatment using bisulfite. The non-methylated cytosine converting treatment using bisulfite is referred to as bisulfite treatment.

When the bisulfite treatment is carried out, the amount (concentration) of bisulfite added is not specifically limited so long as it can sufficiently convert non-methylated cytosine(s) in DNA, and corresponds to, for example, 1M or higher, preferably 1M to 15M, more preferably 3M to 10M as the final concentration in a solution containing DNA. The incubation conditions (temperature and time) after addition of bisulfite may be appropriately selected according to the amount added of bisulfite, and for example, when bisulfite is added at a final concentration of 6M, the incubation is carried out at 50°C to 80°C for 10 minutes to 90 minutes.

Methylation status of CpG sites in DNA can be analyzed by analyzing the sequence of DNA after bisulfite treatment and detecting the difference in base sequence from the original sequence. This process is referred to as bisulfite sequencing method.

Methylation status of CpG sites can be alternatively analyzed by mass spectrometry. Specifically, a DNA after bisulfite treatment as a template is amplified by PCR using a primer set specific for a base sequence which is a target for analysis, and the obtained PCR product is subjected to IVT amplification to convert methylated cytosine and uracil respectively to guanine (G) and adenine (A). The obtained IVT amplification product is digested with RNase A and the difference in mass (16 Da) due to difference between G and A in the obtained digested fragments is detected on a MALDI-TOF (matrix assisted laser desorption/ionization time-of-flight) mass spectrometer to analyze methylation status of DNA. This method is referred to as MassARRAY® analysis.

It is known that the cleavage site in IVT products by RNase A is between an arbitrary base and the adjacent uracil (U) or thymine (T). Thus the base sequence and mass of the IVT product cleaved by RNase A may be predicted based on the base sequence of the template DNA. Accordingly the peaks obtained in MassARRAY® can be identified as to the portions of the base sequences in the template DNA from which the peaks are originated. For example, when one CpG site is methylated in a DNA fragment, a peak obtained in MassARRAY® shifts to the side with an increased mass for 16 Da. When a DNA fragment containing more than one CpG site is analyzed, for example, the DNA fragment having two methylated CpG sites shows a shift of 32 Da and the DNA fragment having three methylated CpG sites shows a shift of 48 Da.

In mass spectrometry such as MassARRAY®, the methylation score of the analyzed DNA fragment can be calculated. For example when a DNA fragment having a certain sequence results in the ratio between the area of the peaks of non-methylated DNA fragments and the area of the peaks of methylated DNA fragments obtained in a resulting chart from the analysis of 1:3, the methylation score of the DNA fragment is 0.75 (= 3/(1+3)). The methylation score is theoretically 1 for a DNA fragment in which all CpG site(s) is methylated and 0 for a DNA fragment without any methylated CpG site.

Methylation status of CpG sites can be analyzed by a methylation specific PCR (MSP) method. The MSP method is a method in which methylation status of CpG sites (presence or absence of methylation) is analyzed by amplifying DNA after bisulfite treatment by PCR using a primer set described hereinafter and determining presence or absence of a PCR product.

The MSP method utilizes a primer set which can amplify a base sequence having a CpG site to be analyzed that is methylated (i.e. cytosine is not converted to uracil) but cannot amplify a base sequence having a CpG site that is not methylated (i.e. cytosine is converted to uracil). According to the MSP method using such a primer set, presence of a PCR product indicates methylation of the CpG site analyzed.

The MSP method may also be carried out by using a primer set which cannot amplify a base sequence having cytosine in a CpG site to be analyzed that is not converted to uracil but can amplify a base sequence having cytosine in a CpG site that is converted to uracil. In this case, absence of a PCR product indicates methylation of the CpG site analyzed.

Each primer in the primer set used for the MSP method may be appropriately designed by a person skilled in the art based on the base sequence including a CpG site to be analyzed, and it is preferably designed so as to contain cytosine of the CpG site to be analyzed at the 3' end or in the vicinity thereof of the primer.

Methylation status of CpG sites may alternatively be analyzed with a microarray. In this case, the microarray for analysis may be prepared by immobilizing a nucleic probe complementary to the base sequence of a promoter region of F13A1 or CHCHD5 gene on a substrate. The microarray can be prepared according to well-known methods in the art.

In the analysis using a microarray, DNA extracted from a biological sample is preferably labeled with a labeling substance well-known in the art. Thus, the present method preferably further comprises the step of labeling the extracted DNA. The labeling step is advantageously carried out after the DNA amplifying step because all DNA in the biological sample may be labeled. The labeling substance may include fluorescence substances, haptens such as biotin, radioactive substances and the like. The fluorescence substances may include Cy3, Cy5, FITC, Alexa Fluor™ and the like. Labeling of DNA facilitates measurement of a signal from a probe on the microarray. A method for labeling DNA with the labeling substance is well-known in the art.

The above signal may be any suitable signal depending on the type of microarrays. For example, the signal may be an electric signal generated when a DNA fragment hybridizes to a probe on the microarray, or a fluorescence or luminescence signal generated from a labeling substance when DNA to be analyzed is labeled as described above. Detection of a signal can be carried out by using a scanner comprised in a conventional microarray analyzer. The scanner may be, for example, GeneChip® Scanner3000 7G (Affymetrix), Illumina® BeadArray Reader (Illumina) and the like.

In the present method, information on uterine body cancer in the subject is obtained based on the analysis result obtained in the analyzing step. The information on uterine body cancer is not particularly limited as far as it may be an index on diagnosis of uterine body cancer and is preferably information indicative of occurrence or status of uterine body cancer or both thereof in a subject. The information preferably includes presence or absence of a cancer cell derived from uterine body cancer in a biological sample collected from a subject. The information may also include, for example, a possibility of occurrence of uterine body cancer in a subject, or a risk for future occurrence of uterine body cancer in a subject. The information on uterine body cancer in a subject who has already been affected by uterine body cancer may include prognosis of the subject, a degree of progression (stage) and the like. The information on uterine body cancer obtained based on the analysis result obtained in the analyzing step does not substantially include information on a type of cancer different from uterine body cancer because the promoter regions of the above genes which are used as markers in the present method are specifically methylated in cancer cells derived from uterine body cancer.

In embodiments of the present method, the analyzing step which provides the analysis result indicating presence of a methylated CpG site may provide information indicating occurrence of uterine body cancer or indicating that the status of uterine body cancer is poor (or aggravated).

In another embodiment of the present method, the above information can be obtained when the methylation frequency obtained in the analyzing step is at or higher than a certain threshold.

More specifically, the information obtained may be indicative of presence of a cancer cell derived from uterine body cancer in a biological sample. The information obtained may alternatively indicate that a subject has a high risk for being affected by uterine body cancer or that a subject has already been affected by uterine body cancer. For a subject who has already been affected by uterine body cancer, information obtained may indicate that prognosis of the subject is poor (or aggravated) or that the cancer is in a progressed stage.

On the contrary, when the result from the analyzing step shows absence of methylated CpG site, information suggesting no occurrence of uterine body cancer or information indicating that uterine body cancer is in a preferable status can be obtained. Alternatively the above information can be obtained when the methylation frequency obtained in the analyzing step is lower than a certain threshold. More specifically, the information obtained may be indicative of absence of a cancer cell derived from uterine body cancer in a biological sample. The information obtained may alternatively indicate that a subject has a low risk for being affected by uterine body cancer or that a subject has not been affected by uterine body cancer. For a subject who has already been affected by uterine body cancer, information obtained may be indicative of a preferable prognosis of the subject or indicate that the cancer is in a relatively early stage.

The threshold is not particularly limited and may be empirically established based on accumulated data on various biological samples. The threshold may alternatively be established as follows. First, methylation frequency is analyzed for DNA extracted respectively from a biological sample which is confirmed to be devoid of cancer cells derived from uterine body cancer (tissues or cells of normal uterine body) and a biological sample containing a cancer cell derived from uterine body cancer. Next, based on the obtained analysis results, a threshold is established within a range that is higher than the methylation frequency of the biological sample devoid of cancer cells and lower than that of the biological sample containing the cancer cell. Preferably, the threshold is established as a value which can highly accurately differentiate between the biological sample devoid of cancer cells and the biological sample containing the cancer cell.

Also disclosed herein is a marker for obtaining information on uterine body cancer by methylation analysis (also merely referred to as "marker"). The marker may be at least one CpG site selected from CpG sites located in promoter regions of F13A1 and CHCHD5 genes.

In embodiments of the present method, methylation status of the herein described marker in a DNA sample prepared from a biological sample collected from a subject may be analyzed and information on uterine body cancer in the subject can be obtained based on the analysis result. The analysis of methylation status and obtainment of information on uterine body cancer are the same as those previously described herein.

The scope of the present invention encompasses the use of a kit in the method for obtaining information on uterine body cancer described herein. The kit comprises a primer set for analysis of methylation status of at least one CpG site selected from CpG sites located in promoter regions of F13A1 and CHCHD5 genes.

The primer set in the kit may be a primer set for analysis of methylation status of CpG sites according to mass spectrometry such as MassARRAY® or an analysis method involving PCR amplification such as the MSP method, the bisulfite sequencing method, among which the primer set for mass spectrometry such as MassARRAY® or for the MSP is preferred. The base sequences of the primers in the primer set may be appropriately selected by a person skilled in the art based on the base sequences in the promoter regions. Examples of the primer set include a primer set of primers having base sequences SEQ ID NOs: 3 and 4; a primer set of primers having base sequences SEQ ID NOs: 5 and 6; a primer set of primers having base sequences SEQ ID NOs: 9 and 10; and a primer set of primers having base sequences SEQ ID NOs: 11 and 12.

Further disclosed herein is a system suitable for provision of information on uterine body cancer in a subject. For example, the system may be as follows.

A system suitable for provision of information on uterine body cancer in a subject comprising a computer containing a processor and a memory controlled by the processor, wherein the memory comprises a computer program for enabling the computer to carry out the steps of:

obtaining an analysis result on methylation status of a CpG site located in a promoter region of at least one gene selected from F13A1 and CHCHD5 in a DNA sample derived from the subject; and
providing information on uterine body cancer in the subject based on the resulting analysis result.

Also disclosed herein is a computer program product for enabling a computer to carry out provision of information on uterine body cancer in a subject. For example, the computer program product may be as follows.

A computer program product for enabling a computer to carry out provision of information on uterine body cancer in a subject comprising a computer readable medium, wherein the medium comprises a computer program for enabling the computer to carry out the steps of:

obtaining an analysis result on methylation status of a CpG site located in a promoter region of at least one gene selected from F13A1 and CHCHD5 in a DNA sample derived from the subject; and
providing information on uterine body cancer in the subject based on the resulting analysis result.

An example of a suitable device for carrying out the present method is illustrated hereinafter by referring to figures. Fig. 7 is a schematic view of an example of a determination device for providing information on uterine body cancer in a subject. The determination device 1 shown in Fig. 7 comprises a measurement device 2 and a computer system 3 connected to the measurement device 2.

In the present example, the measurement device 2 is a MALDI-TOF mass spectrometer. The measurement device 2 obtains mass spectrometric information such as the time of flight or the mass-to-charge ratio (m/z ratio) of a substance to be analyzed. The measurement device 2 onto which a measurement sample prepared from the DNA sample derived from a subject is mounted obtains mass spectrometric information of a nucleic acid in the measurement sample and sends the mass spectrometric information to the computer system 3.

The measurement device 2 may be, when methylation status is analyzed by the MSP method, a gel imaging device such as a fluorescence image scanner. In this case, the measurement device 2 onto which a gel obtained by electrophoresis of a reaction solution after nucleic acid amplification by the MSP method is mounted detects amplification products. The measurement device 2 then obtains the band intensity data of the amplification products and sends the obtained data to the computer system 3.

The computer system 3 comprises a computer main body 3a, an input device 3b and a display 3c which displays specimen information, determination results and the like. The computer system 3 receives the mass spectrometric information from the measurement device 2. The processor in the computer system 3 executes, based on the mass spectrometric information, a program for providing information on uterine body cancer in a subject.

Fig. 8 is a block diagram showing the functionality configuration of the determination device of Fig. 7. As shown in Fig. 8, the computer system 3 comprises a receiving unit 301, a memory unit 302, a calculating unit 303, a determining unit 304 and an output unit 305. The receiving unit 301 is communicably connected to the measurement device 2 though a network. The calculating unit 303 and the determining unit 304 are included in a control unit 306.

The receiving unit 301 obtains information from the measurement device 2. The memory unit 302 stores a threshold necessary for determination and a formula for calculating the methylation score. The calculating unit 303 calculates the methylation score from information obtained at the receiving unit 301 according to the formula stored in the memory unit 302. The determining unit 304 determines whether or not the methylation score calculated at the calculating unit 303 is lower than the threshold stored at the memory unit 302. The output unit 305 outputs the determination result from the determining unit 304 as information on uterine body cancer in the subject (e.g., presence or absence of a cancer cell derived from uterine body cancer in the biological sample collected from the subject).

Fig. 9 is a block diagram showing the hardware configuration of the determination device in Fig. 7. As shown in Fig. 9, the computer main body 3a comprises a CPU (Central Processing Unit) 30, ROM (Read Only Memory) 121, ROM 32, a hard disk 33, an input/output interface 34, a read-out device 35, a communication interface 36 and an image output interface 37. The CPU 30, ROM 31, RAM (Random Access Memory) 32, the hard disk 33, the input/output interface 34, the read-out device 35, the communication interface 36 and the image output interface 37 are connected via a bus 38 so as to be able to communicate with each other.

The CPU 30 can execute a computer program stored in ROM 31 and a computer program loaded with ROM 32. When the CPU 30 executes the application program, the functional blocks described above may be executed. Accordingly the computer system serves as a terminal that is a determination device for providing information on uterine body cancer in a subject.

ROM 31 is made up with mask ROM, PROM, EPROM, EEPROM or the like. ROM 31 stores the computer program executed by the CPU 30 and data used for the execution.

ROM 32 is made up with SRAM, DRAM or the like. ROM 32 is used for readout of the computer programs stored in ROM 31 and the hard disk 33. ROM 32 is also utilized as a work area when the CPU 30 executes these computer programs.

An operating system to be executed by the CPU 30, computer programs such as application programs (the computer program for providing information on uterine body cancer in a subject) and data for executing the computer programs are installed on the hard disk 33.

The read-out device 35 is made up with a flexible disk drive, a CD-ROM drive, a DVD-ROM drive or the like and can read out the computer program or data stored on a portable memory medium 40.

The input/output interface 34 is made up with a serial interface such as USB, IEEE1394 and RS-232C, a parallel interface such as SCSI, IDE and IEEE1284 and an analog interface formed by a D/A converter, an A/D converter or the like. The input/output interface 34 is connected to the input device 3b such as a keyboard and a mouse. A user can input data into the computer main body 3a by means of the input device 3b.

The communication interface 36 is, for example, an Ethernet® interface. The computer system 3 can send printing data to a printer via the communication interface 36.

The image output interface 37 is connected to the display 3c made up with a LCD, a CRT and the like. Accordingly the display 3c can output an image signal according to image data provided by the CPU 30. The display 3c displays an image (on a screen) according to the input image signal.

] The process operations carried out by the determination device 1 for providing information on uterine body cancer in a subject are illustrated hereinafter. Fig. 10 is a flow chart for providing information on uterine body cancer using the determination device of Fig. 7. An example is herein illustrated in which mass spectrometric information of a nucleic acid in a measurement sample prepared from a DNA sample derived from a subject is used for calculation of a peak area from which a methylation score is calculated and the methylation score is determined as to whether or not it is lower than a threshold.

In the step S1-1, the receiving unit 301 in the determination device 1 receives from the measurement device 2 mass spectrometric information. In the next step S1-2, the calculating unit 303 calculates from the mass spectrometric information received at the receiving unit 301 a peak area which is sent to the memory unit 302. In the step SI-3, the calculating unit 303 calculates the methylation score based on the peak area stored in the memory unit 302 according to the formula stored in the memory unit 302.

In the step SI-4, the determining unit 304 determines whether or not the methylation score calculated at the calculating unit 303 is lower than the threshold stored in the memory unit 302. When the methylation score is lower than the threshold, the determining unit 304 in the step S1-5 sends a determination result indicating that the biological sample collected from the subject does not contain a cancer cell derived from uterine body cancer to the output unit 305. When the methylation score is not lower than the threshold (i.e., the methylation score is at or higher than the threshold), the determining unit 304 sends a determination result indicating that the biological sample collected from the subject contains a cancer cell derived from uterine body cancer to the output unit 305.

In the step S1-7, the output unit 305 outputs the determination result as information on uterine body cancer in the subject, so that the display 3c displays the result and/or the printer prints out the result. Accordingly, the determination device can provide, to a physician or the like, information assisting the physician or the like to judge whether or not the subject has uterine body cancer.

The present invention is specifically described hereinafter by way of Examples which do not limit the present invention.

## Examples

Reference Example 1: Identification of novel markers utilizing methylation data of cancerous tissues from uterine body cancer and normal uterine tissue (1) Collection of methylation data

In the present Example, methylation data on the Infinium HumanMethylation27 BeadChip (Illumina) for cancerous tissues of uterine body cancer (60 specimens) and normal uterine tissue (1 specimen) were collected which are published in TCGA (The Cancer Genome Atlas : http://tcga-data.nci.nih.gov/tcga/tcgaHome2.jsp).

(2) Identification of novel markers

The Infinium HumanMethylation27 BeadChip includes probes for methylated and non-methylated CpG sites for each of 27,578 CpG sites on the human genome. In the present Reference Example, the signal intensity (signal M) from probes for methylated CpG sites and the signal intensity (signal U) from probes for non-methylated CpG sites were detected on BeadArray Reader and the methylation rate (mCpG) of CpG sites in the respective genes was calculated according to the following calculation formula:

$$(\mathrm{mCpG}) = (\mathrm{signal\ M})/\{(\mathrm{signal\ M}) + (\mathrm{signal\ U})\}$$

The threshold was set as "0.4" for the methylation rate (mCpG) of genes, and a specimen having a methylation rate at or higher than the threshold was designated as "methylation positive specimen". Based on the number of methylation positive specimens, methylation positive rate (%) for the respective genes in various tissues was calculated according to the following formula:

$$\text{Methylation positive rate (\%)} = (\text{number of methylation positive specimens/total number of specimens}) \times 100$$

For example, for cancerous tissue specimens, the methylation positive rate of a gene can be calculated by "(number of methylation positive specimens among cancerous tissue specimens/total number of cancerous tissue specimens) × 100". The gene which showed a statistically significant difference between cancerous tissue specimens and the normal uterine tissue specimen was identified as a marker which is methylated in cancerous tissues.
As a result of data mining using the Infinium HumanMethylation27 BeadChip (Illumina), promoter regions of F13A1 and CHCHD5 genes were identified as markers which are highly methylated in cancerous tissues of uterine body cancer (see Figs. 1A and 1B). These markers may also be referred to as the present markers hereinbelow.
Reference Example 2: Comparison of methylation data between cancer/tumor tissue specimens derived from several types of cancer/tumor, non-cancerous tissue specimens and normal tissue specimens

(1) Collection of methylation data

In the present Reference Example, in addition to the cancerous tissues of uterine body cancer (60 specimens) and normal uterine tissue (1 specimen) described in Reference Example 1, methylation data of 9 types of cancer/tumor tissue specimens, 6 types of non-cancerous tissue specimens and 13 types of normal tissue specimens were compared. The number of specimens for the respective tissues is shown in the following tables.

Table 2

| **Cancer/tumor tissue specimens** | |
|---|---|
| **Tissue** | **No. of specimens** |
| **Brain tumor (Brain)** | 283 |
| **Colon cancer (Colon)** | 236 |
| **Breast cancer (Breast)** | 186 |
| **Lung cancer (Lung)** | 59 |
| **Gastric cancer (Gastric)** | 82 |
| **Hepatocellular carcinoma (Liver)** | 100 |
| **Renal cell carcinoma (Kidney)** | 219 |
| **Ovarian cancer (Ovary)** | 519 |
| **Prostate cancer (Prostate)** | 93 |

Table 3

| **Non-cancerous tissue** | |
|---|---|
| **Tissue** | **No. of specimens** |
| **Colonic mucosa (Colon)** | 16 |
| **Lung** | 59 |
| **Cirrhosis tissue (Liver)** | 39 |
| **Kidney** | 199 |
| **Ovary** | 16 |
| **Prostate** | 87 |

Table 4

| **Normal tissue** | |
|---|---|
| **Tissue** | **No. of specimens** |
| **Normal brain (Brain)** | 2 |

(continued)

| Normal tissue | |
|---|---|
| Tissue | No. of specimens |
| Normal oral mucosa (Oral) | 2 |
| Normal lung (Lung) | 2 |
| Normal colonic mucosa (Colon) | 2 |
| Normal liver (Liver) | 2 |
| Peripheral blood from healthy subject (Blood) | 2 |
| Normal skeletal muscle (Skeletal) | 2 |
| Normal testis (Testis) | 1 |
| Normal gastric mucosa (Stomach) | 2 |
| Normal pancreas (Pancreas) | 1 |
| Normal spleen (Spleen) | 1 |
| Normal kidney (Kidney) | 2 |
| Peripheral blood from healthy subject (Blood) * (Blood, Sahia B, et al.) | 93 |

Among the above specimens, the methylation data for cancerous tissue specimens derived from gastric cancer, cancerous tissue specimens derived from hepatocellular carcinoma, non-cancerous tissue specimens derived from hepatocellular carcinoma, normal tissue specimens derived from normal liver and 12 types of normal tissue specimens were those measured by Infinium Methylation Assay using the Infinium HumanMethylation27 BeadChip (Illumina) in the same manner as described in Reference Example 1. The methylation data for other specimens were the methylation data of the Infinium HumanMethylation27 BeadChip (Illumina) available from TCGA (The Cancer Genome Atlas: http://tcga-data.nci.nih.gov/tcga/tcgaHome2.jsp) or, for the specimens of lung cancer and prostate cancer and 93 specimens of peripheral blood from healthy subjects, published in the following references. The methylation data in this context are the methylation rate (mCpG) of CpG sites in F13A1 and CHCHD5 obtained as described in section (2) in Reference Example 1.

- Lung cancer: Selamat SA et al., Genome-scale analysis of DNA methylation in lung adenocarcinoma and integration with mRNA expression. Genome Res. Jul; 22 (7): 1197-211 (2012).
- Prostate cancer: Kobayashi Y et al., DNA methylation profiling reveals novel biomarkers and important roles for DNA methyltransferases in prostate cancer. Genome Res. Jul 21 (7): 1017-27 (2011).
- Data of 93 specimens of peripheral blood from healthy subjects: Salhia B et al., DNA methylation analysis determines the high frequency of genic hypomethylation and low frequency of hypermethylation events in plasma cell tumors. Cancer Res. Sep 1; 70 (17): 6934-44 (2010).

(2) Comparison of methylation positive rate between cancer/tumor tissue specimens derived from several types of cancer/tumor, non-cancerous tissue specimens and normal tissue specimens

The threshold was set as "0.4" for the methylation rate (mCpG) of genes, and a specimen having a methylation rate at or higher than the threshold was designated as "methylation positive specimen". Based on the number of methylation positive specimens, methylation positive rate (%) for the present markers in various tissues was calculated according to the above formula. The results are shown in Figs. 2A and 2B. In Fig. 2, "Normal tissue" represents, among the tissues indicated in Table 4, the normal tissues excluding 93 specimens of peripheral blood from healthy subjects (Salhia B et al.) and "Normal blood" represents the 93 specimens of peripheral blood from healthy subjects (Salhia B et al.).

Figs. 2A and 2B show that both of the present markers are not highly methylated in other types of cancer or human normal tissues or human normal blood (peripheral blood from healthy subjects, Salhia B et al.) and thus are specifically and highly methylated in uterine body cancer.

Comparative Example 1: Comparison of methylation positive rate between cancer/tumor tissue specimens derived from several types of cancer/tumor, non-cancerous tissue specimens and normal tissue specimens

The methylation positive rate was calculated for each of MLH1 and CDKN2A genes (hereinafter referred to as "known markers") which have already been known to be methylated in cancer cells derived from uterine body cancer in the similar manner as Reference Example 2 in the respective tissues. The results are shown in Figs. 3A and 3B.

According to Figs. 3A and 3B, MLH1 and CDKN2A showed high positive rates in uterine body cancer and were also methylated in other types of cancer and other non-cancerous tissues. Therefore, these genes have low specificity towards uterine body cancer. Thus, the known markers have high sensitivity while having low specificity as markers of uterine body cancer, thereby posing issues in terms of performance as diagnostic markers of uterine body cancer. Namely it was shown that a search for markers is required to be carried out by taking the specificity in other types of cancer and other non-cancerous tissues into account.

Example 1: Comparison of methylation data (MassARRAY) between normal uterus and uterine body cancer

(1) Biological samples

In the present Example, the biological samples used were normal uterine tissues (4 specimens) and cancerous tissues of uterine body cancer (4 specimens).

(2) Preparation of measurement samples and control samples

(i) Extraction of genomic DNA

Genomic DNA was extracted from the above tissues with the QIAamp DNA Mini Kit (QIAGEN). The genomic DNA contained in the obtained DNA solution was fragmented with Bioruptor (COSMO BIO). In order to generate a calibration curve for mass spectrometry, the control genomic DNA used was genomic DNA of human peripheral blood lymphocytes. The genomic DNA from human peripheral blood lymphocytes was amplified with the GenomiPhi v2DNA amplification kit (GE Healthcare Life Sciences). The obtained amplification product consisted of non-methylated DNA. The amplification product was fragmented with Bioruptor (COSMO BIO) to obtain a solution of non-methylated DNA fragments (0% methylated DNA). A portion of the solution of the non-methylated DNA fragments was subjected to reaction with SssI methylase (New England Biolab) to methylate all cytosines in CG sequences and obtain a solution of methylated DNA fragments (100% methylated DNA). By mixing the 0% methylated DNA solution and the 100% methylated DNA solution at certain proportions, solutions of 25%, 50% and 75% methylated DNA were obtained.

(ii) Bisulfite treatment

The respective DNA fragments (500 ng) obtained as above were subjected to bisulfite treatment with the EZ DNA Methylation Kit (Zymo Research) and the treated genomic DNA was eluted in sterilized distilled water (80 μl).

(iii) Amplification by PCR and IVT

Methylated cytosine and uracil in the DNA fragments after bisulfite treatment were converted respectively to guanine and adenine by PCR and IVT amplification.

It is verified by MassARRAY® analysis using control samples as described hereinbelow that the primer sets used for PCR can amplify both methylated DNA and non-methylated DNA without bias. The sequences of the primer sets for the present markers are shown in Table 5. The base sequences (sequences of negative strands) which can be analyzed with the primer sets shown in Table 5 in the promoter regions in F13A1 and CHCHD5 genes are shown in SEQ ID NOs: 13 and 14, respectively.

Table 5

| Marker | Base sequence of primer | | SEQ ID NO: |
|---|---|---|---|
| *F13A1* | **Forward** : | AGGAGAAAATTTTGTGGGATA | 3 |
| | **Reverse :** | CAAAATCRATAACTTACCTACAAAC | 4 |
| *CHCHD5* | **Forward :** | TAGATTATTGGGTAAAAGAA | 5 |
| | **Reverse :** | AAAACCAAACTAAACTAAAC | 6 |

The following tag sequence and T7 promoter sequence were respectively added to the 5'-terminals of the forward primers and reverse primers in the above primer sets for IVT reaction.

- Tag sequence: AGGAAGAGAG (SEQ ID NO: 7)
- T7 promoter sequence: CAGTAATACGACTCACTATAGGGAGAAGGCT (SEQ ID NO: 8)

PCR reaction solution was prepared by mixing the following reagents:

| | |
|---|---|
| 10 × Hot Star buffer (QIAGEN) | 0.5 μL |
| 25 mM dNTP mix | 0.04 μL |
| Hot Star Taq (5 U/μL) (QIAGEN) | 0.04 μL |
| Primer mix | 2.0 μL |
| DNA solution | 1.0 μL |
| Water | 1.42 μL |
| Total | 5 μL |

PCR reaction was carried out on the reaction solution under the following conditions:

1 cycle of 94°C for 15 minutes;

45 cycles of 94°C for 20 seconds, 52°C for 30 seconds and 72°C for 1 minute; and 72°C for 3 minutes.

The obtained PCR products were dephosphorylated with SAP (Shrimp Alkaline Phosphatase) in the MassCLEAVE™ Reagent kit (SEQUENOM). The following reaction solution prepared with the kit was then added.

| | |
|---|---|
| 5 × T7 R&DNA polymerase buffer | 0.89 μL |
| T Cleavage mix | 0.24 μL |
| 100 mM DTT | 0.22 μL |
| T7 R&DNA polymerase | 0.44 μL |
| RNase A | 0.06 μL |
| RNase-free water | 3.15 μL |
| Total | 5 μL |

The obtained mixture was incubated at 37°C for 3 hours to effect IVT reaction and uracil or thymine specific cleavage. The obtained reaction product was purified with Clean Resin (SEQUENOM) to obtain a sample for mass spectrometry. Samples derived from genomic DNA extracted from the above clinical specimens were designated as measurement samples and samples derived from control genomic DNA were designated as control samples, both of which were used for mass spectrometry described hereinbelow.

(3) Analysis of methylation status by mass spectrometry using MassARRAY®

(i) Preparation of calibration curve

Two mass spectrometric analyses were carried out independently on the control samples obtained as above. On the basis of the obtained analysis results calibration curves were prepared for the respective primer sets and correlation coefficients were calculated. Accordingly it was verified that the primer sets used could amplify both methylated DNA and non-methylated DNA without bias.

(ii) Analysis of measurement samples

The measurement samples obtained as above were subjected to mass spectrometry to obtain peaks of DNA fragments contained in the measurement samples. The obtained peaks were then allocated to the portions of the base sequences of F13A1 and CHCHD5. The methylation score was calculated on the basis of the ratio between the area of the peaks of the fragments containing a methylated CpG site and the area of the peaks of the fragments without the methylated CpG site, both fragments having the same base sequence. The obtained results are shown in Fig. 4A and 4B.

According to Figs. 4A and 4B, it was found that F13A1 and CHCHD5 showed low methylation scores in normal uterine tissues and significantly high methylation scores in uterine body cancer. It was therefore demonstrated that the methylation score of the present markers correlates with uterine body cancer, similar to the results of the Infinium method in Reference Example 1. In addition, according to Fig. 4, it was found that analysis of methylation frequency of the present markers allows establishment of the threshold that can distinguish specimens of uterine body cancer and specimens of normal uterine tissues.

Example 2: Comparison of methylation data (MSP) between normal uterine tissue and uterine body cancer

(1) Biological samples

In the present Example, the biological samples used were normal uterine tissue (1 specimen) and cancerous tissues of uterine body cancer (4 specimens).

(2) Preparation of measurement samples and control samples

(i) Extraction of genomic DNA

Genomic DNA was extracted from the above tissues with the QIAamp DNA Mini Kit (QIAGEN). The genomic DNA contained in the obtained DNA solution was fragmented with Bioruptor (COSMO BIO).

The control genomic DNA used was genomic DNA of human peripheral blood lymphocytes. The genomic DNA from human peripheral blood lymphocytes was amplified with the GenomiPhi v2DNA amplification kit (GE Healthcare Life Sciences). The obtained amplification product consisted of non-methylated DNA. The amplification product was fragmented with Bioruptor (COSMO BIO) to obtain a solution of non-methylated DNA fragments (0% methylated DNA). A portion of the solution of the non-methylated DNA fragments was subjected to reaction with SssI methylase (New England Biolab) to methylate all cytosines in CG sequences and obtain a solution of methylated DNA fragments (100% methylated DNA).

(ii) Bisulfite treatment

The respective DNA fragments (500 ng) obtained as above were subjected to bisulfite treatment with the EZ DNA Methylation Kit (Zymo Research) and the treated genomic DNA was eluted in sterilized distilled water (80 μl).

(3) Methylation specific PCR (MSP)

MSP was carried out on the measurement samples and control samples (DNA after bisulfite treatment) obtained in the above section (2). The composition of PCR reagents, primer sets and reaction conditions for PCR in MSP are shown below.

| | |
|---|---|
| DDW (sterilized water) | 16.8 μL |
| 10 × PCR buffer with $MgCl_2$ (Roche) | 2.5 μL |
| 2 mM dNTP mix | 2.5 μL |
| 10 μM sense primer | 1.0 μL |
| 10 μM antisense primer | 1.0 μL |
| Faststart Taq polymerase (Roche) | 0.2 μL |
| Measurement sample or control sample | 1.0 μL |
| Total | 25 μL |

<Primer set>

The primer sets used for MSP are shown in Table 6. These primer sets allow generation of amplification products when DNA in the amplified regions is methylated. A primer set for accuracy control was also used which allows judgment on whether or not the bisulfite treatment had been appropriately carried out (see Table 7). The base sequences (sequences of negative strands) which can be analyzed with the primer sets shown in Table 6 in the promoter regions in F13A1 and CHCHD5 genes are shown in SEQ ID NOs: 17 and 18, respectively.

Table 6

| Gene symbol | Primer | Base sequence of primer | SEQ ID NO: | Size of PCR product (bp) | Annealing temp. (°C) | Cycles |
|---|---|---|---|---|---|---|
| *F13A1* | F13A 1_MF | TAATTGTTTTGTTTTTTTCGAGTTC | 9 | 109 | 54 | 36 |
| | F13A1_MR | AATCGATAACTTACCTACAAAGGCT | 10 | | | |
| *CHCHD5* | CHCHD5_MF | ATTTATATTTCGAGATTTTTGTCG | 11 | 100 | 56 | 36 |
| | CHCHD5_MR | AACCAAACTAAACTAAACGCCG | 12 | | | |

Table 7

| Base sequence of primer | | SEQ ID No: | Size of PCR product (bp) | Annealing temp. (°C) | Cycles |
|---|---|---|---|---|---|
| **Forward** | GGGATATTAAGTGGAGTTATTTTGGTTTTAGTT | 15 | 129 | 60 | 40 |
| **Reverse** | CCCTCCCAACATCCTTCCTAA | 16 | | | |

<PCR reaction conditions>

95°C for 6 minutes;

Y cycles of 95°C for 30 seconds, X°C for 30 seconds and 72°C for 30 seconds; 72°C for 7 minutes; and

keep at 16°C.

In the above reaction conditions, "X" and "Y" respectively represent the annealing temperature and the number of cycles as indicated in Tables 6 and 7.

(4) Analysis of results of methylation specific PCR (MSP)

The amplification product from MSP was verified by 2% agarose gel electrophoresis. The results are shown in Fig. 5. In this figure, "0" and "100" under "control" represent the 0% methylation control sample and the 100% methylation control sample, respectively.

In PCR using the primer set for accuracy control, bands were detected in all lanes as shown in Fig. 5. This shows that bisulfite treatment of the samples was appropriately carried out. In PCR using the present markers, bands derived from methylated CpGs were not detected for normal uterine tissue. In contrast, PCR of uterine body cancer tissues resulted in detection of bands in 3 samples among 4 samples for both markers of F13A1 and CHCHD5. Accordingly it is indicated that in methylation analysis of the present markers by MSP method, methylation of the present markers and uterine body cancer are correlated, similar to the result of the Infinium method from Reference Example 1. Namely it is suggested that F13A1 and CHCHD5 are markers with high specificity such that they tend to be highly methylated in uterine body cancer but methylation thereof is not detected in other normal tissues.

Example 3: Methylation analysis by MSP using blood samples

In the present Example, methylation analysis was carried out by MSP for CpG sites in the promoter region of F13A1 using peripheral blood as a biological sample.

(1) Biological samples

In the present Example, the biological samples used were peripheral blood collected from patients with uterine body cancer (5 specimens). The control samples used were peripheral blood from healthy subjects (3 specimens).

(2) Preparation of measurement samples

(i) Extraction of genomic DNA

Plasma was obtained from each of the above peripheral blood (2 ml) according to the standard method. Genomic DNA was extracted from the resulting plasma with the QIAamp Circulating Nucleic Acid Kit (QIAGEN).

(ii) Bisulfite treatment

The respective DNA fragments (500 ng) obtained as above were subjected to bisulfite treatment with the EZ DNA Methylation Kit (Zymo Research) and the treated genomic DNA was eluted in sterilized distilled water (80 μl).

(3) MSP

Qualitative MSP was carried out with the measurement samples obtained in the above section (2). The composition of PCR reagents and conditions for PCR are shown below. The base sequences of the primer sets used in the present Example (F13A1 and accuracy control) are shown in Tables 6 and 7.

<PCR reagent>

| | |
|---|---|
| DW (sterilized water) | 10.88 μL |
| 10 × PCR buffer with $MgCl_2$ (Roche) | 1.5 μL |
| 10 mM dNTP mix | 0.3 μL |
| 10 μM sense primer | 0.6 μL |
| 10 μM antisense primer | 0.6 μL |
| Faststart Taq polymerase (Roche) | 0.12 μL |
| Measurement sample | 1.0 μL |
| Total | 15 μL |

<PCR reaction conditions>

95°C for 6 minutes;
50 cycles of 95°C for 30 seconds, X°C for 30 seconds and 72°C for 30 seconds; 72°C for 7 minutes; and keep at 16°C.

In the above reaction conditions, "X" represents annealing temperature which is 54°C for F13A1 and 60°C for the primer set for accuracy control.

(4) Analysis of results of MSP

The amplification product from MSP was verified by 2% agarose gel electrophoresis. The band intensities of amplification products were represented in graphs (see Figs. 6A and 6B). The bars in Fig. 6A indicated under peripheral blood of healthy subjects correspond to the background, indicating absence of detection of any band. According to Fig. 6A, it is found that PCR for F13A1 allowed detection of no band in peripheral blood from healthy subjects and detection of bands in 3 samples among 5 samples of peripheral blood from uterine body cancer patients. Fig. 6B shows that bands were detected in all samples by PCR with the primer set for accuracy control. This indicates that the bisulfite treatment of the samples had been appropriately carried out. Accordingly it is found that the present marker tends to be highly methylated in uterine body cancer patients and methylation thereof is not detected in healthy subjects even when the biological sample used is peripheral blood. Therefore it is suggested that the present marker is highly specific for uterine body cancer and is useful for determining presence or absence of cancer cells derived from uterine body cancer in blood samples.

Reference Signs List

1 Determination device
2 Measurement device
3 Computer system
3a Computer main body
3b Input device
3c Display

## Claims

1. A method for obtaining information on the presence or absence of a cancer cell derived from uterine body cancer in a biological sample collected from a subject comprising the steps of:

   preparing a DNA sample from the biological sample collected from the subject;
   analyzing methylation status of a CpG site in a promoter region of at least one gene selected from F13A1 and CHCHD5 in the DNA sample obtained from the preparation step; and
   obtaining information on the presence or absence of a cancer cell derived from uterine body cancer in the biological sample collected from the subject based on an analysis result obtained from the analyzing step.

2. The method according to claim 1, wherein the analyzing step is the step of analyzing presence or absence of methylation of at least one CpG site.

3. The method according to claim 2, wherein
   the step of obtaining information is the step of obtaining information indicating that the biological sample contains a cancer cell derived from uterine body cancer when the analysis result shows that there is a methylated CpG site.

4. The method according to claim 1, wherein the analyzing step is the step of analyzing methylation frequency.

5. The method according to claim 4, wherein
   the step of obtaining information is the step of obtaining information indicating that the biological sample contains a cancer cell derived from uterine body cancer when the methylation frequency obtained from the analyzing step is higher than a predetermined threshold.

6. Use of a kit in a method for obtaining information on the presence or absence of a cancer cell derived from uterine body cancer in a biological sample collected from a subject according to claim 1, said kit comprising a primer set for analysis of methylation of at least one CpG site selected from CpG sites located in promoter regions of F13A1 and CHCHD5 genes.

7. The use according to claim 6, wherein the primer set is a primer set for analyzing methylation status of the CpG site by at least one method selected from mass spectrometry and methylation specific PCR method.

8. The use according to claim 7, wherein the primer set is at least one selected from:

   a primer set of primers respectively having base sequences SEQ ID NOs: 3 and 4;
   a primer set of primers respectively having base sequences SEQ ID NOs: 5 and 6;
   a primer set of primers respectively having base sequences SEQ ID NOs: 9 and 10; and
   a primer set of primers respectively having base sequences SEQ ID NOs: 11 and 12.

## Patentansprüche

1. Verfahren zum Gewinnen von Informationen bezüglich des Vorliegens oder Fehlens einer von Gebärmutterkrebs abgeleiteten Krebszelle in einer von einem Individuum entnommenen biologischen Probe, umfassend die folgenden Schritte:

   Herstellen einer DNA-Probe aus der von dem Individuum entnommenen biologischen Probe;

Analysieren des Methylierungszustands einer CpG-Stelle in einer Promoterregion von mindestens einem Gen, ausgewählt aus F13A1 und CHCHD5, in der von dem Herstellungsschritt erhaltenen DNA-Probe; und
Gewinnen von Informationen über das Vorliegen oder Fehlen einer von Gebärmutterkrebs abgeleiteten Krebszelle in der von dem Individuum entnommenen biologischen Probe aufgrund eines von dem Analysierschritt erhaltenen Analyseergebnisses.

**2.** Verfahren nach Anspruch 1, wobei es sich bei dem Analysierschritt um den Schritt des Analysierens des Vorliegens oder Fehlens der Methylierung von mindestens einer CpG-Stelle handelt.

**3.** Verfahren nach Anspruch 2, wobei:
es sich bei dem Schritt des Gewinnens von Informationen um den Schritt des Gewinnens von Informationen, die anzeigen, dass die biologische Probe eine von Gebärmutterkrebs abgeleitete Krebszelle enthält, wenn das Analyseergebnis zeigt, dass eine methylierte CpG-Stelle vorliegt, handelt.

**4.** Verfahren nach Anspruch 1, wobei es sich bei dem Analysierschritt um den Schritt des Analysierens der Methylierungsfrequenz handelt.

**5.** Verfahren nach Anspruch 4, wobei:
es sich bei dem Schritt des Gewinnens von Informationen um den Schritt des Gewinnens von Informationen, die anzeigen, dass die biologische Probe eine von Gebärmutterkrebs abgeleitete Krebszelle enthält, wenn die von dem Analysierschritt erhaltene Methylierungsfrequenz höher als eine vorbestimmte Schwelle ist, handelt.

**6.** Verwendung eines Kits in einem Verfahren zum Gewinnen von Informationen bezüglich des Vorliegens oder Fehlens einer von Gebärmutterkrebs abgeleiteten Krebszelle in einer von einem Individuum nach Anspruch 1 entnommenen biologischen Probe, wobei das Kit einen Primer-Satz für die Analyse der Methylierung von mindestens einer CpG-Stelle, ausgewählt aus CpG-Stellen, die sich in den Promoterregionen der F13A1- und CHCHD5-Gene befinden, umfasst.

**7.** Verwendung nach Anspruch 6, wobei es sich bei dem Primer-Satz um einen Primer-Satz zum Analysieren des Methylierungszustands der CpG-Stelle mit mindestens einem Verfahren, ausgewählt aus Massenspektrometrie und methylierungsspezifischem PCR-Verfahren, handelt.

**8.** Verwendung nach Anspruch 7, wobei der Primer-Satz mindestens einer ausgewählt aus den folgenden ist:

ein Primer-Satz von Primern, die die Basensequenzen SEQ ID Nos: 3 bzw. 4 aufweisen;
ein Primer-Satz von Primern, die die Basensequenzen SEQ ID Nos: 5 bzw. 6 aufweisen;
ein Primer-Satz von Primern, die die Basensequenzen SEQ ID Nos: 9 bzw. 10 aufweisen; und
ein Primer-Satz von Primern, die die Basensequenzen SEQ ID Nos: 11 bzw. 12 aufweisen.

## Revendications

**1.** Procédé destiné à obtenir des informations sur la présence ou l’absence d’une cellule cancéreuse dérivée d’un cancer du corps de l’utérus dans un échantillon biologique prélevé sur un sujet, comprenant les étapes consistant à :

préparer un échantillon d’ADN à partir de l’échantillon biologique prélevé sur le sujet ;
analyser l’état de méthylation d’un site CpG dans une région promotrice d’au moins un gène choisi parmi les F13A1 et CHCHD5 dans l’échantillon d’ADN obtenu à partir de l’étape de préparation ; et
obtenir des informations sur la présence ou l’absence d’une cellule cancéreuse dérivée d’un cancer du corps de l’utérus dans l’échantillon biologique prélevé sur le sujet, sur la base d’un résultat d’analyse obtenu à partir de l’étape d’analyse.

**2.** Procédé selon la revendication 1, dans lequel l’étape d’analyse est l’étape d’analyse de la présence ou de l’absence d’une méthylation d’au moins un site CpG.

**3.** Procédé selon la revendication 2, dans lequel l’étape d’obtention d’informations est l’étape d’obtention d’informations qui indiquent que l’échantillon biologique contient une cellule cancéreuse dérivée d’un cancer du corps de l’utérus quand le résultat d’analyse montre qu’il y a un site CpG méthylé.

**4.** Procédé selon la revendication 1, dans lequel l'étape d'analyse est l'étape d'analyse de la fréquence de méthylation.

**5.** Procédé selon la revendication 4, dans lequel
l'étape d'obtention d'informations est l'étape d'obtention d'informations qui indiquent que l'échantillon biologique contient une cellule cancéreuse dérivée d'un cancer du corps de l'utérus quand la fréquence de méthylation obtenue à partir de l'étape d'analyse est supérieure à un seuil prédéterminé.

**6.** Utilisation d'une trousse dans un procédé destiné à obtenir des informations sur la présence ou l'absence d'une cellule cancéreuse dérivée d'un cancer du corps de l'utérus dans un échantillon biologique prélevé sur un sujet selon la revendication 1, ladite trousse comprenant un ensemble d'amorces destiné à l'analyse d'une méthylation d'au moins un site CpG, choisi parmi des sites CpG situés dans des régions promotrices des gènes F13A1 et CHCHD5.

**7.** Utilisation selon la revendication 6, dans laquelle l'ensemble d'amorces est un ensemble d'amorces destiné à analyser l'état de méthylation du site CpG par le biais d'au moins un procédé choisi parmi la spectrométrie de masse et un procédé d'ACP spécifique d'une méthylation.

**8.** Utilisation selon la revendication 7, dans laquelle l'ensemble d'amorces est au moins un élément choisi parmi :

un ensemble d'amorces ayant respectivement les séquences de bases SEQ ID n° : 3 et 4 ;
un ensemble d'amorces ayant respectivement les séquences de bases SEQ ID n° : 5 et 6 ;
un ensemble d'amorces ayant respectivement les séquences de bases SEQ ID n° : 9 et 10 ; et
un ensemble d'amorces ayant respectivement les séquences de bases SEQ ID n° : 11 et 12.

Fig. 1A

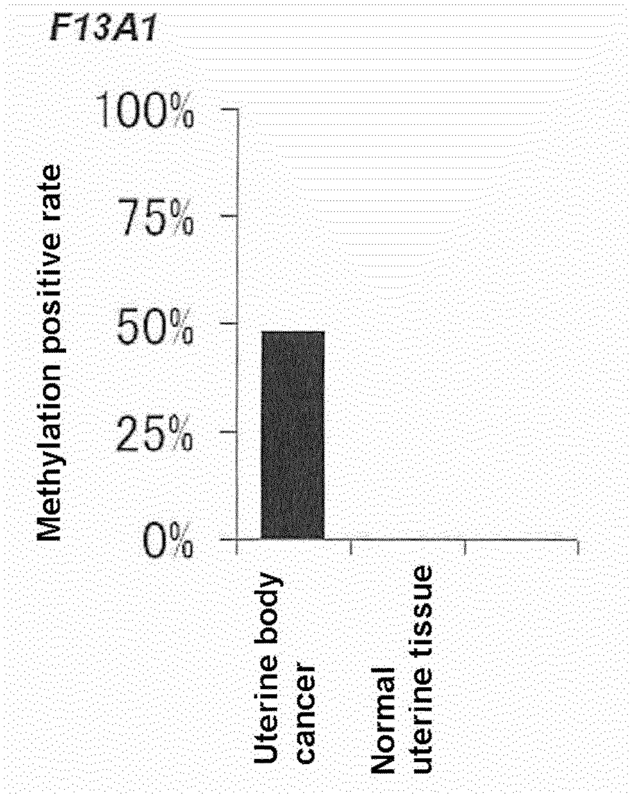
F13A1
Methylation positive rate
100%
75%
50%
25%
0%
Uterine body cancer
Normal uterine tissue

Fig.1B

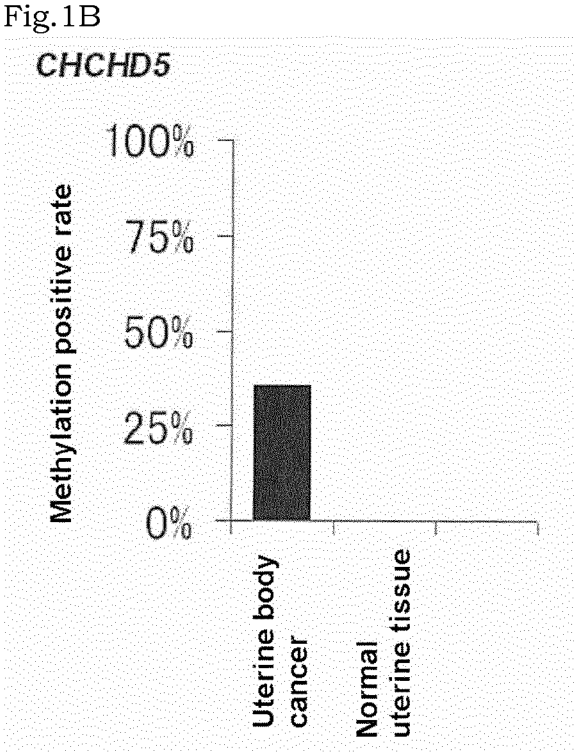
CHCHD5
Methylation positive rate
100%
75%
50%
25%
0%
Uterine body cancer
Normal uterine tissue

Fig. 2A

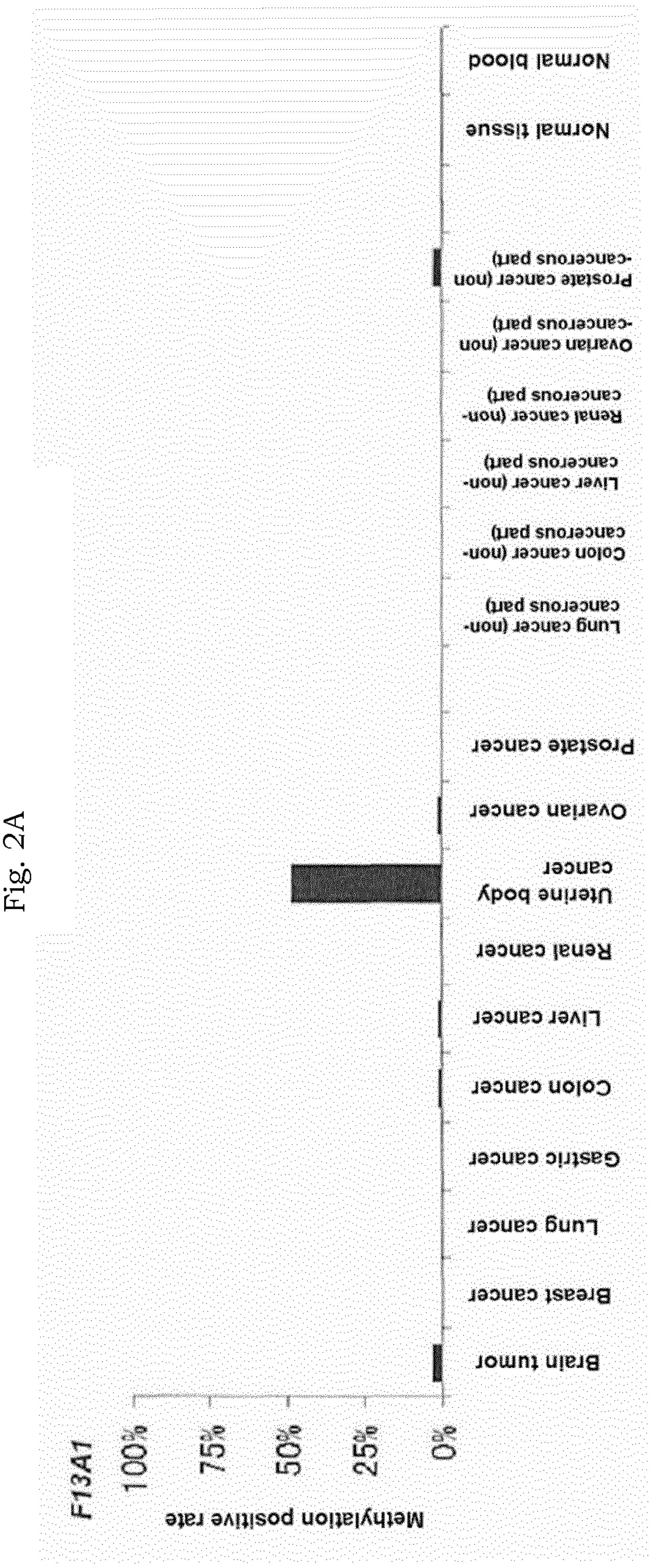
F13A1
Methylation positive rate
100%
75%
50%
25%
0%
Brain tumor
Breast cancer
Lung cancer
Gastric cancer
Colon cancer
Liver cancer
Renal cancer
Uterine body cancer
Ovarian cancer
Prostate cancer
Lung cancer (non-cancerous part)
Colon cancer (non-cancerous part)
Liver cancer (non-cancerous part)
Renal cancer (non-cancerous part)
Ovarian cancer (non-cancerous part)
Prostate cancer (non-cancerous part)
Normal tissue
Normal blood

Fig. 2B

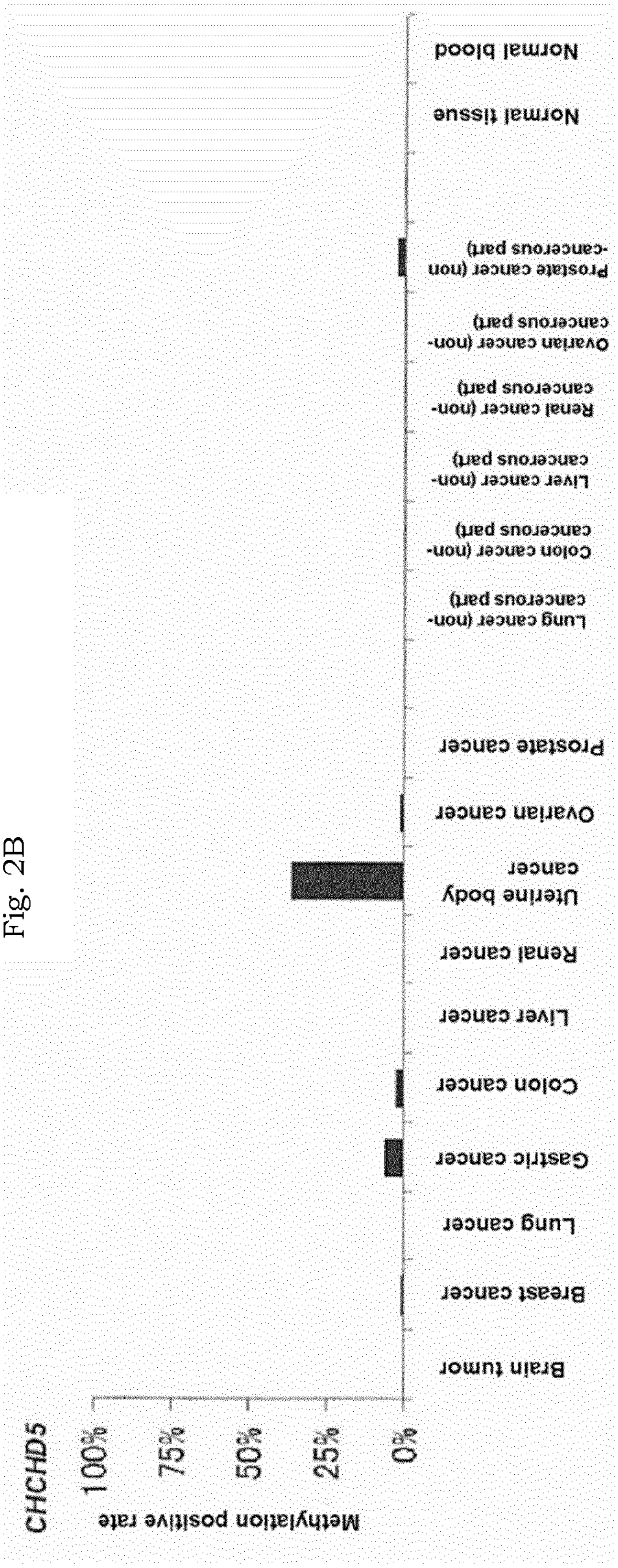
CHCHD5
Methylation positive rate
100%
75%
50%
25%
0%
Brain tumor
Breast cancer
Lung cancer
Gastric cancer
Colon cancer
Liver cancer
Renal cancer
Uterine body cancer
Ovarian cancer
Prostate cancer
Lung cancer (non-cancerous part)
Colon cancer (non-cancerous part)
Liver cancer (non-cancerous part)
Renal cancer (non-cancerous part)
Ovarian cancer (non-cancerous part)
Prostate cancer (non-cancerous part)
Normal tissue
Normal blood

Fig. 3A

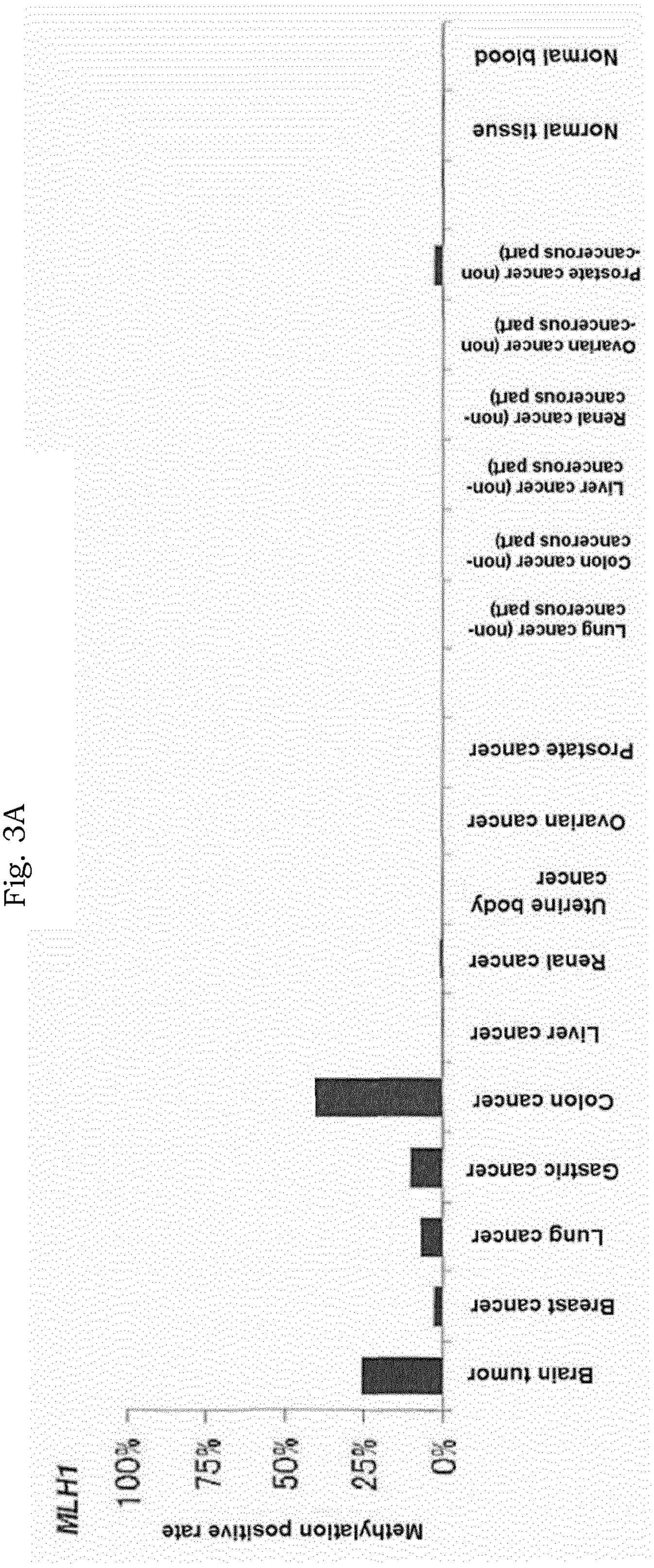
MLH1
Methylation positive rate
100%
75%
50%
25%
0%
Brain tumor
Breast cancer
Lung cancer
Gastric cancer
Colon cancer
Liver cancer
Renal cancer
Uterine body cancer
Ovarian cancer
Prostate cancer
Lung cancer (non-cancerous part)
Colon cancer (non-cancerous part)
Liver cancer (non-cancerous part)
Renal cancer (non-cancerous part)
Ovarian cancer (non-cancerous part)
Prostate cancer (non-cancerous part)
Normal tissue
Normal blood

Fig. 3B

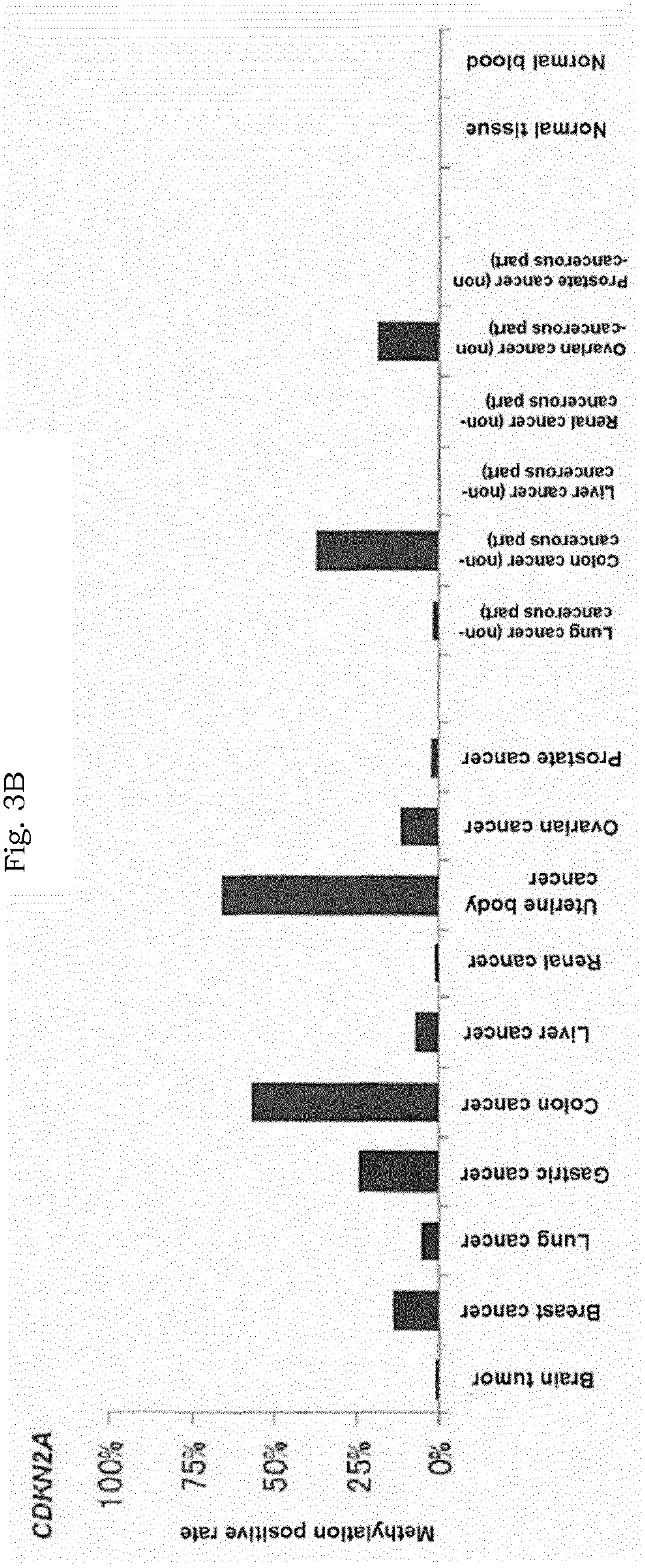
CDKN2A
Methylation positive rate
100%
75%
50%
25%
0%
Brain tumor
Breast cancer
Lung cancer
Gastric cancer
Colon cancer
Liver cancer
Renal cancer
Uterine body cancer
Ovarian cancer
Prostate cancer
Lung cancer (non-cancerous part)
Colon cancer (non-cancerous part)
Liver cancer (non-cancerous part)
Renal cancer (non-cancerous part)
Ovarian cancer (non-cancerous part)
Prostate cancer (non-cancerous part)
Normal tissue
Normal blood

Fig. 4A

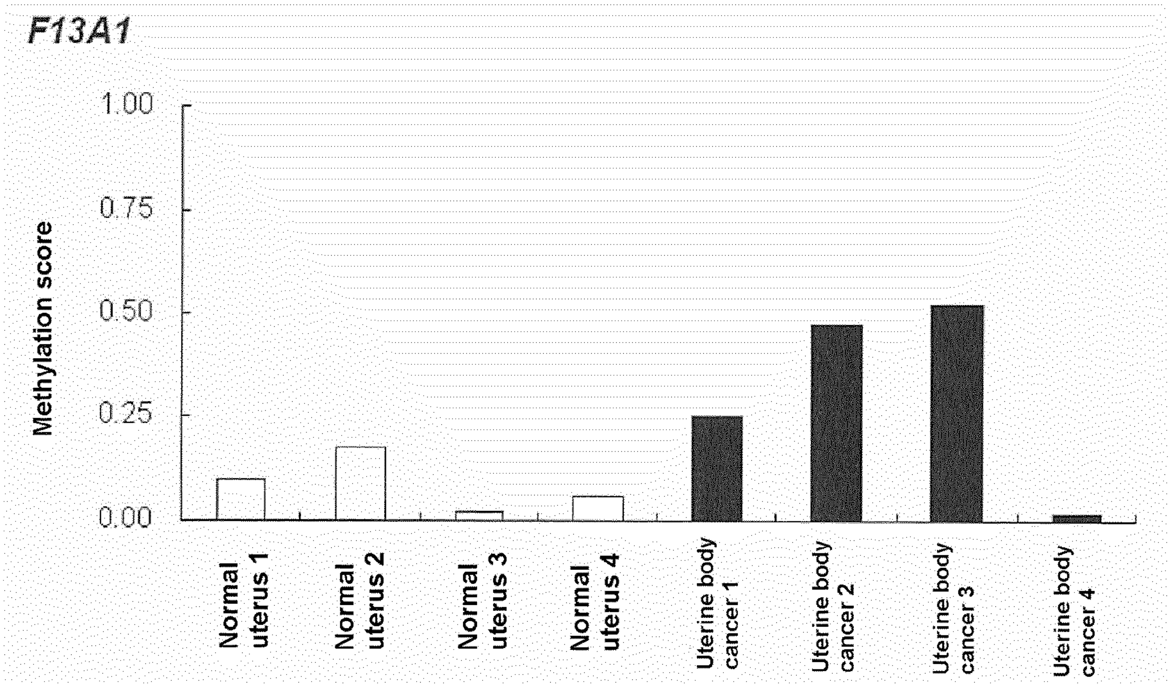
F13A1
Methylation score
1.00
0.75
0.50
0.25
0.00
Normal uterus 1
Normal uterus 2
Normal uterus 3
Normal uterus 4
Uterine body cancer 1
Uterine body cancer 2
Uterine body cancer 3
Uterine body cancer 4

Fig. 4B

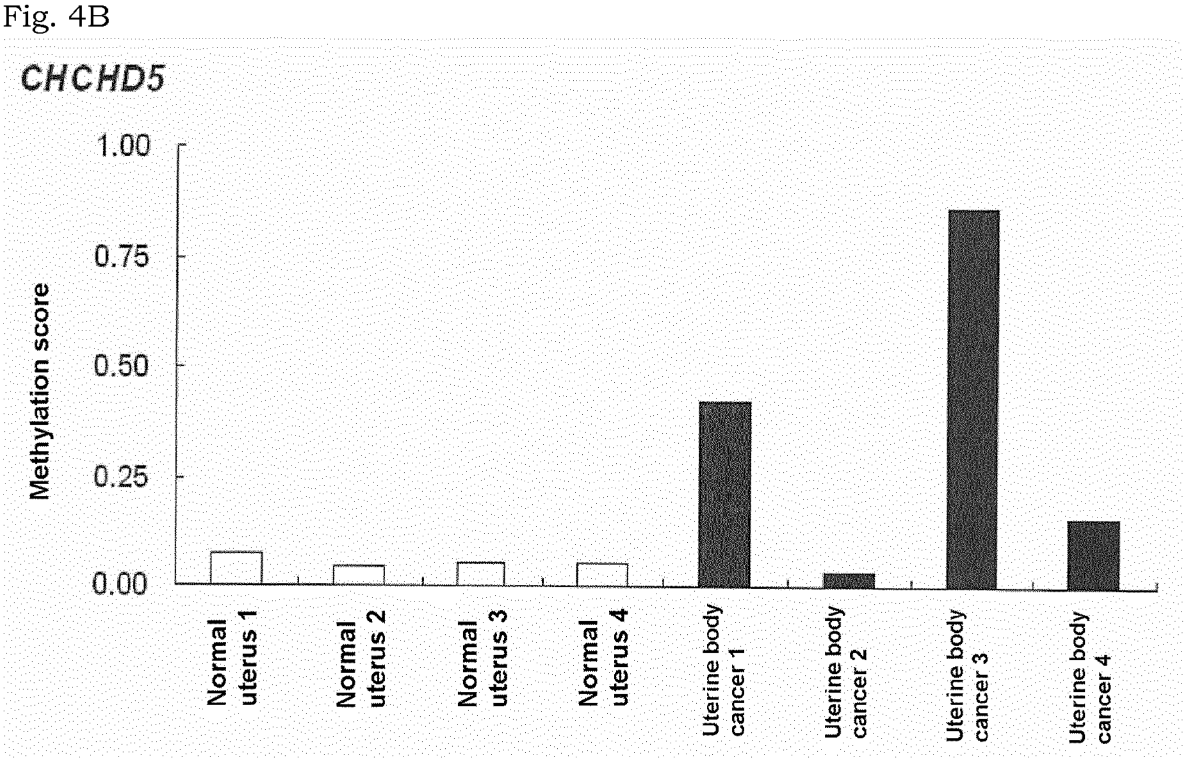
CHCHD5
Methylation score
1.00
0.75
0.50
0.25
0.00
Normal uterus 1
Normal uterus 2
Normal uterus 3
Normal uterus 4
Uterine body cancer 1
Uterine body cancer 2
Uterine body cancer 3
Uterine body cancer 4

Fig. 5

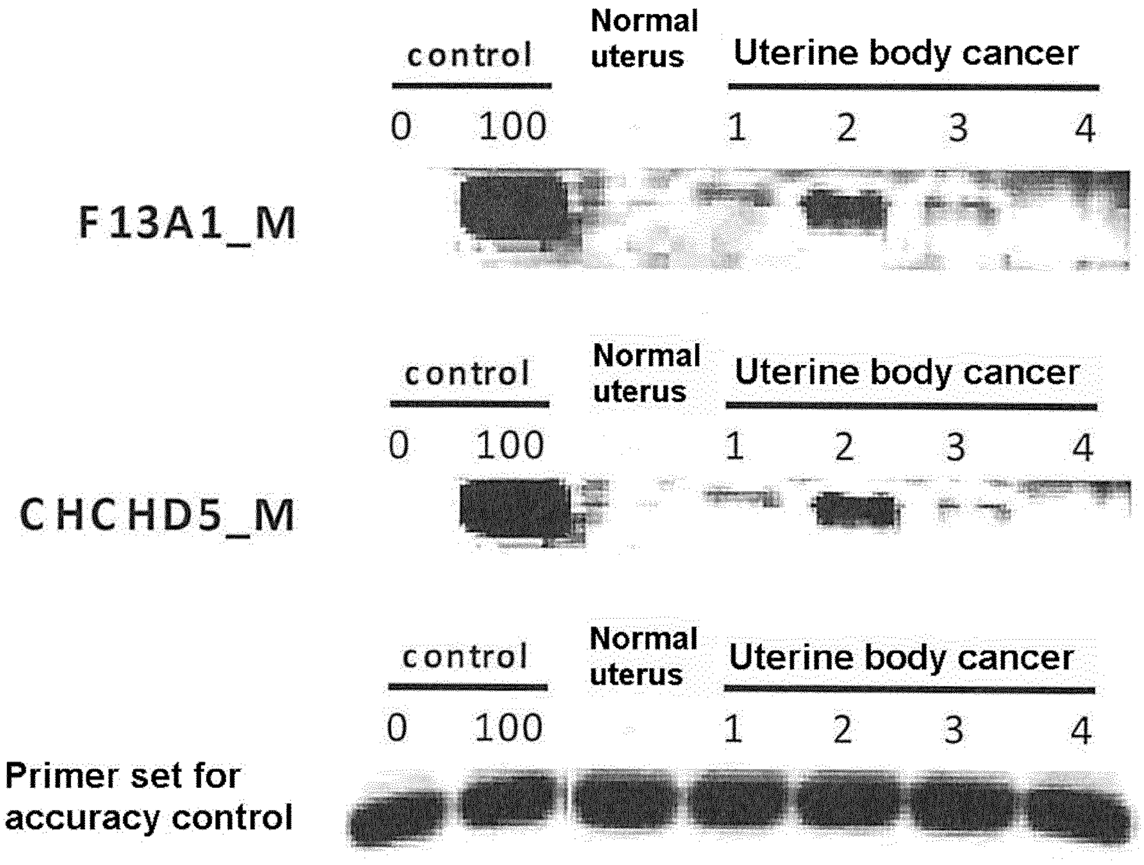
control
Normal uterus
Uterine body cancer
0
100
1
2
3
4
F13A1_M
control
Normal uterus
Uterine body cancer
0
100
1
2
3
4
CHCHD5_M
control
Normal uterus
Uterine body cancer
0
100
1
2
3
4
Primer set for accuracy control

Fig. 6A

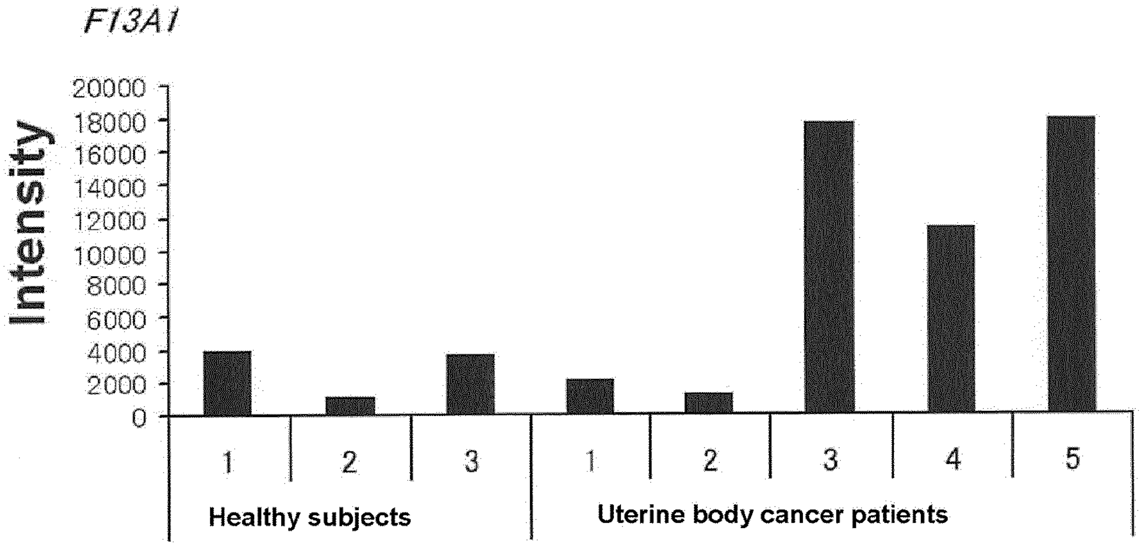
F13A1
Intensity
20000
18000
16000
14000
12000
10000
8000
6000
4000
2000
0
1
2
3
1
2
3
4
5
Healthy subjects
Uterine body cancer patients

Fig. 6B

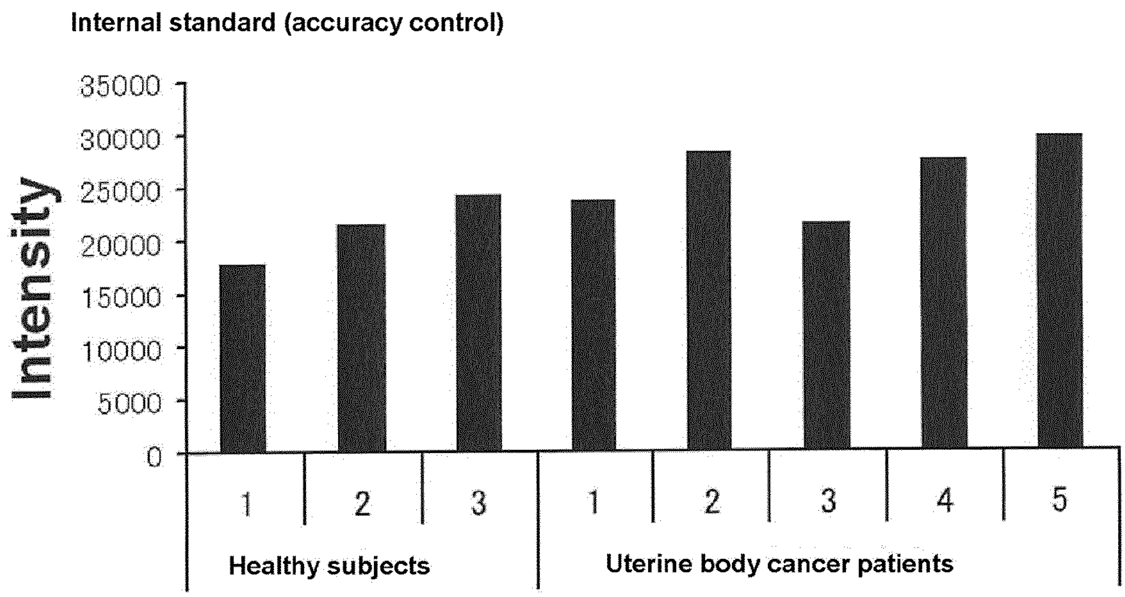
Internal standard (accuracy control)
Intensity
35000
30000
25000
20000
15000
10000
5000
0
1
2
3
1
2
3
4
5
Healthy subjects
Uterine body cancer patients

Fig. 7

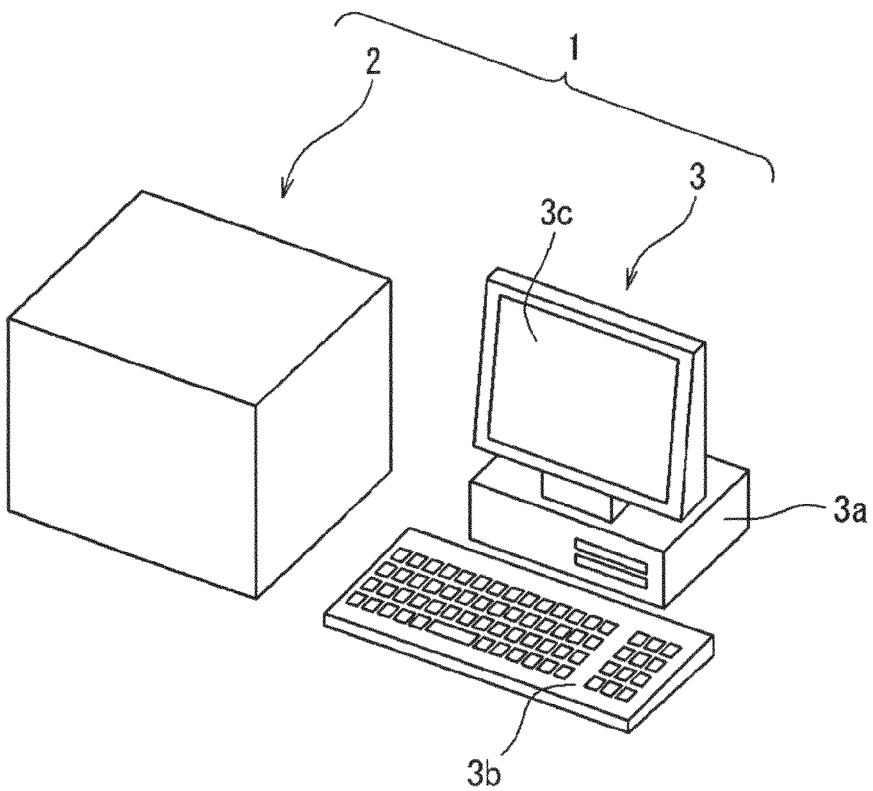
1
2
3
3c
3a
3b

Fig. 8

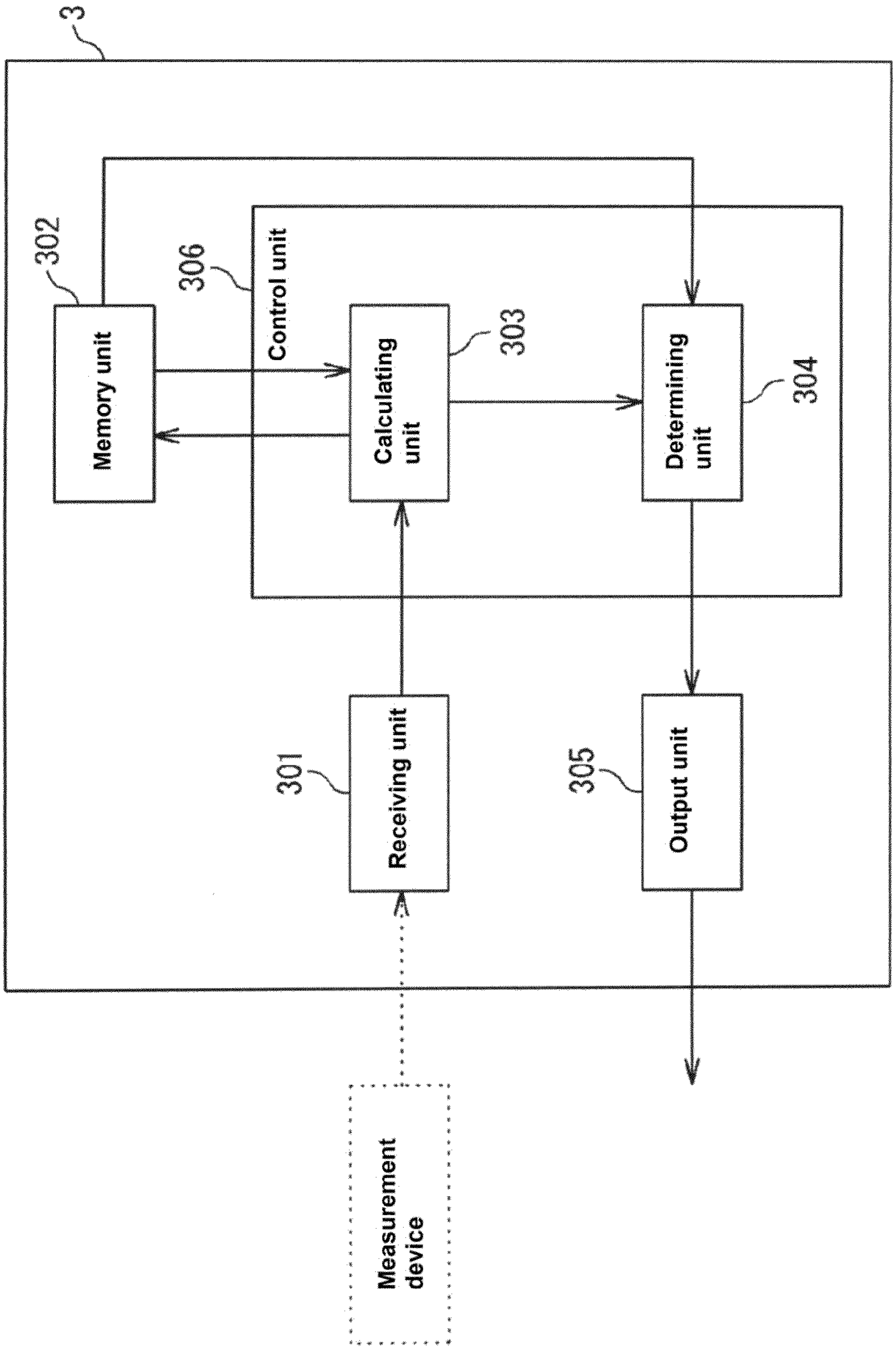
3
302
Memory unit
306
Control unit
Calculating unit
303
Determining unit
304
301
Receiving unit
305
Output unit
Measurement device

Fig. 9

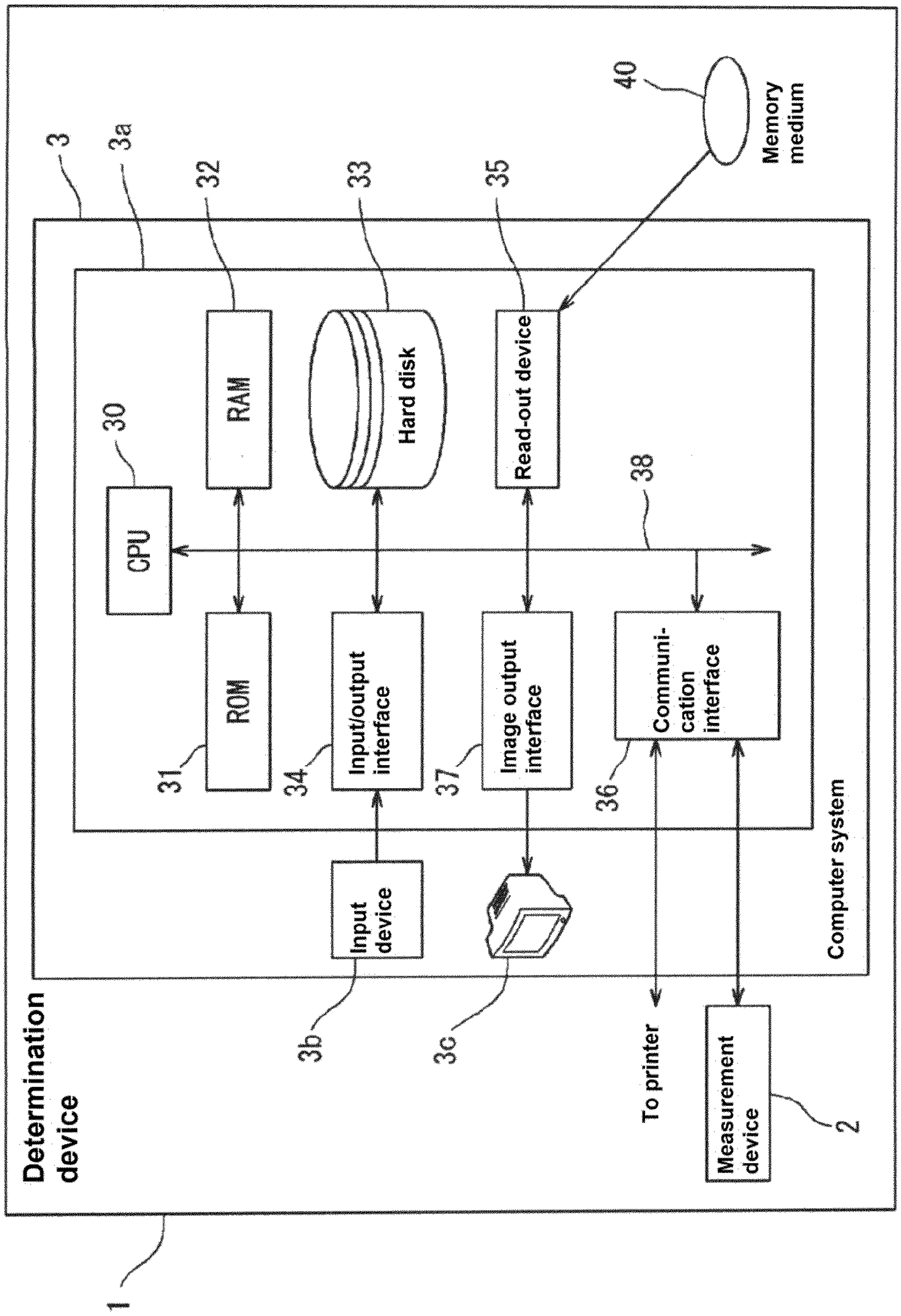
Determination device
1
3
3a
CPU
30
RAM
32
ROM
31
Hard disk
33
Read-out device
35
Memory medium
40
38
Input/output interface
34
Image output interface
37
Communication interface
36
Input device
3b
3c
Computer system
To printer
Measurement device
2

Fig. 10

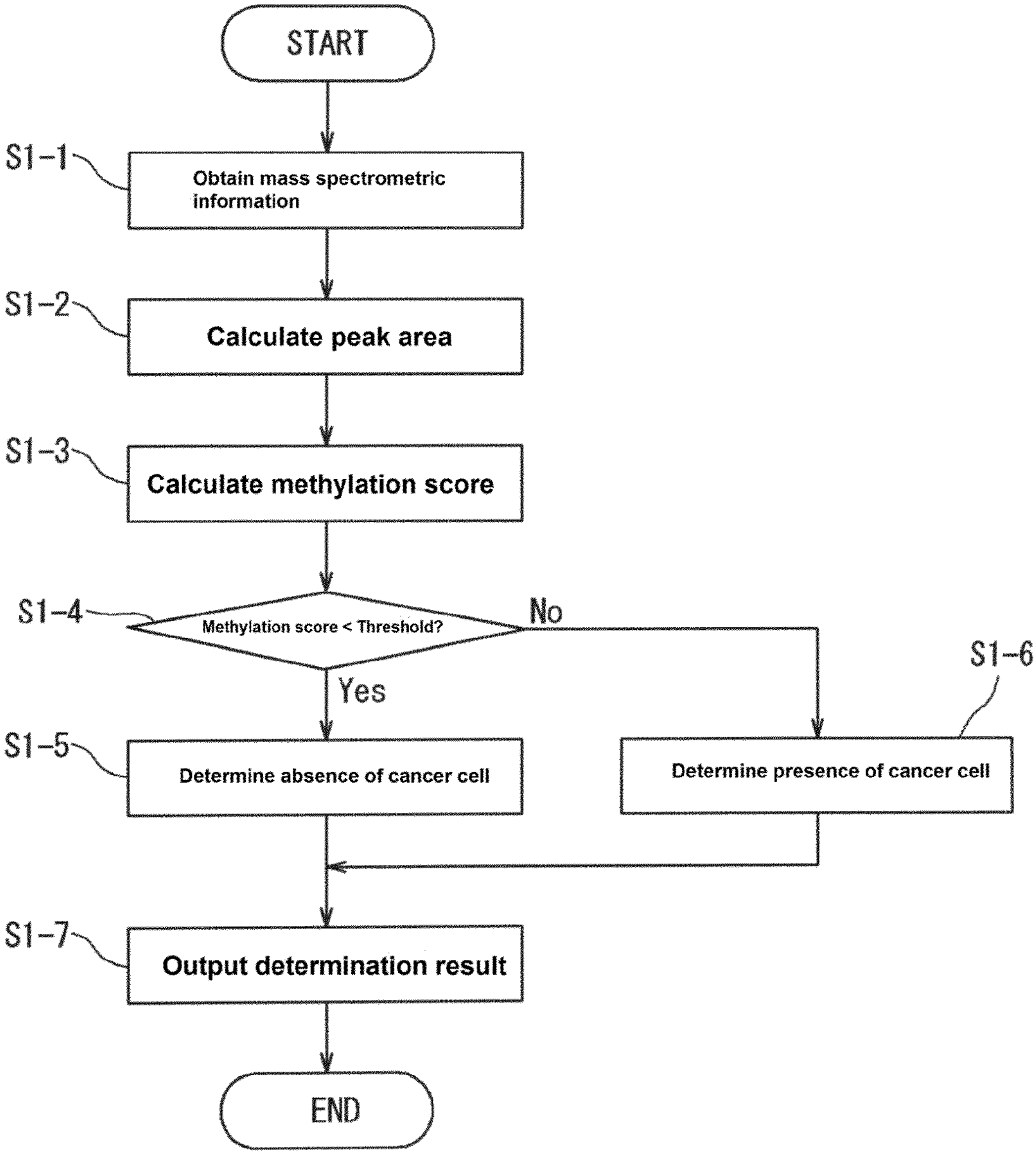
START
S1-1
Obtain mass spectrometric information
S1-2
Calculate peak area
S1-3
Calculate methylation score
S1-4
Methylation score < Threshold?
No
Yes
S1-5
Determine absence of cancer cell
S1-6
Determine presence of cancer cell
S1-7
Output determination result
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012205769 A


### Non-patent literature cited in the description


- **ESTELLER M. et al.** *Am. J. Pathol.,* 1996, vol. 155, 1767-1772
- **FURLAN D. et al.** *Clin. Cancer Res.,* 2006, vol. 12, 3329-3336
- **KOLBE et al.** *Plos One,* 2012, vol. 7 (3), e32941
- **SELAMAT SA et al.** Genome-scale analysis of DNA methylation in lung adenocarcinoma and integration with mRNA expression. *Genome Res.,* July 2012, vol. 22 (7), 1197-211
- **KOBAYASHI Y et al.** DNA methylation profiling reveals novel biomarkers and important roles for DNA methyltransferases in prostate cancer. *Genome Res.,* July 2011, vol. 21 (7), 1017-27
- **SALHIA B et al.** DNA methylation analysis determines the high frequency of genic hypomethylation and low frequency of hypermethylation events in plasma cell tumors. *Cancer Res.,* 01 September 2010, vol. 70 (17), 6934-44

SEQUENCE LISTING

<110> SYSMEX CORPORATION
THE UNIVERSITY OF TOKYO

<120> METHOD FOR OBTAINING INFORMATION ON CANCER OF UTERINE BODY, AND MARKER AND KIT FOR OBTAINING INFORMATION ON CANCER OF UTERINE BODY

<130> TM5780PC

<150> JP2012-205769
<151> 2012-09-19

<160> 18

<170> PatentIn version 3.5

<210> 1
<211> 4176
<212> DNA
<213> Homo sapiens

<400> 1

```
ggattttagc cctgaaatgc tcattctgac tgcactgtag agaatggact ggaaaggggt     60
acatacacgg gtagattcaa gggctgttta agaggtagga tagaaaatat ttgctcaagg    120
atcggatttg atgattgagg gagagaaaca aggaagcctc aagaatgatg cccatgtgtc    180
tggtgtacca cctgcgtgca aagtgatggt attcattgag gtagggaact cctgagatgc    240
accaggtttt ccaggaaagt gatacatctc atgttagaca tgatgagcgt gtgttgtcac    300
tgggatgtga caaatgagga tagccagtag gcacttagat acgggaatct gcaattctgg    360
aaaagatctt ggctaaagac ccagatttgg gaggtgtcag gatatagatg gaatttgaaa    420
actatgggag ttcctcagac aatcaaggaa tattgcgctg cactggagga aaccacagag    480
tgttcaatgt caaggaggcc taggaaaaat accttcaaag acagagttca atgcttccaa    540
aaggtcaggt aaataagaac tgaatcctgt cttttggcct gagcacaggc cttggagacc    600
gtgacctcca ctttcagtag aatgatgagg cggaagccag atggcagata cttagtgatg    660
aaggggagga gaggaggagt aacagcagga gtggggacat aggttggggg aggtttttaa    720
aaaaatactg aagagacctg aaattgcatg aatgctgaga agacccacta ttagcaagca    780
ggagcttgaa gctgcaagag gaagagtacg taagagtcac agcaaagccc cggagaacaa    840
ggcaacgcag aagcctggtt tggagatgca gagggaggag ggacctttcc aacttcagta    900
acaagaagaa agaggaaaag atagataagg ttgcaggcag gttcatagat tggtagcaga    960
aaattgagag aatttctaac caatggcatc tttttttttc ttctttataa cattgggaga   1020
ttgagtggca gggtcagaaa tttgaagaca gtaaagaagg ttagaaatag ccatgtttga   1080
ctgggtgcgg tggctcacgc ctgtaatccc aacactttgg gaagccaagg agggtggatc   1140
acctgaggtc agaagttcga gaccagcctg gccaacatgg taaagcccca tctctactaa   1200
```

aaatacaaaa attaggcggg catggtggca tgcacctgta gttccagcta cttgggaggc 1260
tgaggcagaa gaatcacttg aacccgggag gtggaggttg cactccagcc tttgcaacag 1320
agcaagactt catctgaaag atagaaagat gaaagaaaga aagaaagaaa gagtaaaaga 1380
aaaaaattaa aattttaggg ggaaaatttt ctaatttttg aacatgcact aaaatgattt 1440
tcagagaaaa ccaagtgtta ttttctaatc tgcatggcat tattaaagat gtttactcat 1500
cttccttggg gctaggcatc ccattcctgc aggaagtctt gtggttaggc ggtggctgtg 1560
gctctgggat gattcaggaa tgcagaccat gccttatttg cacaggaggg acacgttgct 1620
ctcactgcat gcatggtcac aactgtctgt gtgacctgtc tgtgggaggg cagacccccc 1680
tcctgccaag ccacagtagc agctgacctg ccaggcaaaa gcattccttt gtaaaggaaa 1740
ccctcccaga ccctctgacc aaaggggacg ggtggggact tcctggcact gcagggcccc 1800
ttgcctggta ctcccagcaa ctggttgcgg tggggagggc agatctaggg atggggatgg 1860
ggaggagaag tgggaatggg aaattgggag tcagaggggc aagtgggaga gaagggcgca 1920
cctgccggga taacaggcca gatgaagtaa atagaaaatc ctctgagctc ccctactggc 1980
tccagctgtg gagaaggggg aggagaaaac cctgtgggac aggggaggag ggtgagggct 2040
cctcttagga agttatttaa gagccaactg tcttgtcttt cccgagtccg tttgaggaag 2100
tccccgaggc gcacagagca agcccacgcg agggcacctc tggaggggag cgcctgcagg 2160
taagccaccg accctgcacc ctatgagcca gggcccgctg cgtccacctt ctgcacctcg 2220
gtttcctggt tgaaccagca agcggcttgc tctgggccct gtggcgccgg tcacaggcag 2280
ctccacttgc ccaatcctgg cttcccgccc ccaactccgc acctgcccag cccagcttct 2340
aagcgggact tctctcaggc tctcgaggca gctctcccga agccgttgct tgcttagaat 2400
gccaccaaat ggggatgggg gagttggagg tgcttcccca agggacgagc cccctttcct 2460
cagggagcag attatggagc tggaacacaa aaggtggagg gagtggggcc aagtgagaac 2520
agcctgctag actggaaccc cgccggcttg gctccccact cctgcgcgcc tccccactcc 2580
tgcgcatcgc ctgcctccgt cccaatttgg agctgaactc gagccacttc tcctccttag 2640
acccgcccag aagcgagggc tcctgcccgc cgcactcacc cagccaggcg tgcacctggg 2700
gctccccccct tctccccctt agcactgtgt ctcccgacag cgcgccacac tgttctgcac 2760
ccctggcaat gccgcagcct accttcggta tttctcacta gaggatggct ctcgcagcct 2820
tctccctatc attaaccctt tttcgctctt ctccctccct tcccccgtca gctcaccaat 2880
gactgtaaat atatttatct atacttatgg atacccattc acaccttatg catccaaaca 2940
ctcctgtctc cttctcagac cagacacctt aggcgtggtt gtcttttctc cagcatgttg 3000
ttagtgctcc acatgtctct ggaaccatct ctctactctg gtggcctcta taatggggac 3060

tcttcacctc ttgggggtgt gcctggtttt tgaaaatagc caagtctcca ggagtcatga 3120
ctgggtaatc caggggatca agggggaacc ttcactgggc ccagaaacaa agtcccctt 3180
catttggtca tttggtcaac aagtacttac tgggcactga ccgtcaggtg tgtgttgttt 3240
aaactggctc agtgcctgca caagctccaa gggaagggga agcaggcttg gggaatggga 3300
aatgttggca accctgactt cttaagccta ttgatagccc tattgaaaat tgcatgccca 3360
acctctccac ctccttatcc tcctccatgt ggctcaattt ccaaagcagt tcctacctcc 3420
tcacccctaa gtggatttac ttatggctct tcttgggctc caccctaacc ctatcagact 3480
ataatctatc agagcaaaca ttctgttttg ttctctgatg tatcccatgt acctagatca 3540
cagcctggca tgtcataggc cctcaataaa tattggttga attaaattgc aaatgtaaac 3600
gctttagaag acaacatttg tctggacatg gttgtgtgcg tttattctta aaagtgactt 3660
ataatctcat aaatctgtat gaaatgaaca tagatacagc acatgcatat aacaggaaac 3720
atttcttgcg aagatgctgc tgtaggatta tggagggtgg gggaatctgg gtctccaaaa 3780
atcttttaat ttttaaattt ctttgaataa gccccttttc ttctgctttc catatgtttt 3840
gacacataca aaaatctccc caagatcctt ggggaactgt attccatcat tagactaatc 3900
cttgctgcca cttctcagtt tttatttatg caaacggcaa aatgtgttgc tcaagtgcta 3960
tcacacacag atatatctgt ttctctattt tggaatcctt gtctcaaatg ttactcactt 4020
tacatgcctt ttctgttgtc ttcttttttt ttttttctg aaggaccttg taaagtcaaa 4080
aatgtcagaa acttccagga ccgcctttgg aggcagaaga gcagttccac ccaataactc 4140
taatgcagcg gaagatgacc tgcccacagt ggagct 4176

<210> 2
<211> 4159
<212> DNA
<213> Homo sapiens

<400> 2

```
acacaaagcc actggagagc tttaagcata ggtgtgatga gaccagattt cagtccttac        60
atgctcaccc aggccgcttt atggaggcaa gaaaagaggg aggaagggag accagggaga       120
aggctggggc aggtatcctg gaggtggagg gtgagggtgg tggtggatta aagcaggggc       180
tatatggggc aagaagtgat gatgttctgg attctggatg catggcaaag gcctgtgtgg       240
tgatggggtg gggctgagga tgactccaaa gtctttaccc tgaacaacca gaaggatgaa       300
ggggatgtta tggagaaaag agatgaactg aggtgagccc ctcttacctc cacagttgca       360
ggtgagcgtg gcggctgcac ctgctcagcg cactgcagga agcggcgcat atgctctgca       420
cagttgccca cagctgcctc gttctgtcga agacactcct cgaaggcctc aaaaggctga       480
gcacaggcct ggcggatctg gcggatgatt gggctgtggg ggtagggcag ttcagcccct       540
```

gggagattcc caccccactg cccacccccc acactgtctc atacttgccc gcactcactg 600
ggaggatgtg cactgggcaa tgctcatctt aaggtagtga cagtcccgct gccaggattc 660
cggcttggcc gccacacact ggccatactg ctccagctcc cggccacagt agcgagcggt 720
gacctctagg gccgcctgcc tgtgggacag gatgggctga ggatcattcc aggcagtagg 780
caagcaaggg aattcttttt tttttttta tttttagag acagggtctc actgtggccc 840
aggctggagt gcagtggcac gatcatagct cactgtagcc accaactcct ggattcaagt 900
gatcttcctg ccccaacctc ctaagtagct gggaccacag gcatgagcca ccatgcccag 960
ctaattttt atgttttgta gagacagggt cttgctatgt tgccgaggct ggtattgaac 1020
ccctgggctc aagcgatccc cctgcttaga cctcccaaag tgctaggatt acaggcataa 1080
gctatggtgc tccgcaaaga aagggagttc tggtctgggt ctgaagtagg actaggagtt 1140
cagggcagat gtgggaatct gaaggatcct gagatggtgc caaagaagtg gagaggtgag 1200
gcccagccct ggggcttgta aagcaggagg ggagcttctg gggagagagt ggaggttcta 1260
aaacaaagtc tgattaatgt aaggtaggcc ttcagggtcc tagaagacag gccttgaaat 1320
ccaggcatcc cccagtaggc ctggggatca tgcacagaag acttggggcc ttagaagaaa 1380
gatctagaat tccagggaga gcttgagagc ccttaatggg tttgggggtt ctaaaagtga 1440
ggtttagagc tccaagaggt ggggtctcag gtcctgggga agagtgtctt agccaaagag 1500
tcttcaagat gaaacctaag gccctggtgc gggactcaag gtcccagggg cgtgaagtct 1560
ggaagccctg gatgaggttg gtgctctggg aatcctgggc aaggtcagag tggtctagga 1620
acaagtctgt ggaccctata ggtgaggact ggggtcccag gcagatctgg aaggttcaaa 1680
aggtaaagtt tggtgatcaa gggggaggaa cctggagctc tgaggatggg tgtaggagtc 1740
atagaggtcc ctaaataagt ctgtagtctt aggatttcag tgtgaaggcc ggacctgggg 1800
tactgggggc agagattggg tgaagagtga ctggaccgaa gtcctaagca gcgcgagagg 1860
gggcgaggcc taaacgctta gagggcgggg cttgggcatg cggagggttg gagctcgtga 1920
gcgaaggcgc tgaatcctga gggtcagatc tccaagaagg agggaggctg gtcctagttc 1980
ccgaggtcct agactaggtc tagatcactg ggtaaaagaa ggggagcggc agcacgtatg 2040
gggtaggcgc tctcactact cacatctcga gacctttgcc ggcgtagggc tgtccggggg 2100
gaacgacccg ccttttccgg tatcggttgt catggcggcg cccagcccag cctggttttt 2160
tccggtagcc aattgaacta acaaccccgt tccctttagg actaatctgt cacggcggcg 2220
ccctacctct tcttccggcc cggctcgtca tggcggcgcc ctgagtagcc acttccgccc 2280
tcttctggct aaattgcttc atccctattt ccggcttgcc atggcagcgc tcggggttta 2340
acggaagtaa gcaatggaaa gtatggtggt gcctcgggtc caaagagaat gcgccgctga 2400
gttgcgcggc acctgacggg attgagcctc agacacaagc gctccaaatc tccgactgta 2460

gctggtggga agcgcctgct gttgctgcag cgtctcaaca tgttgttccc attttcgttg 2520

ggtgctgtag cggtaggctg ttgggctcag ggataggggg cctgattcct aacgccgcgc 2580

tgccaccatc ggccttgcgc agacgctgat aatccccctg gcacccaggt tcatgcgagc 2640

gcttggaaca ggagggaaga gggagtcaaa ccaagagccg aactaattcg gcttgggcct 2700

gccatttacc cagcctggga cccagacgtt tccccatccc ttagcgtacg ccaggacaga 2760

catggcgttt aatcctgact ccatgagact atcaagctag ttacttcctc tctttgtaat 2820

taattcctcc taatgtagca acatttcccc cttccttctc atctgtctta aaagtgtgag 2880

gttagacctg ggcgtgagtt tccccacata agagactgga tatcccctgg tcagagaact 2940

gagcatcagt cctgccgttt cgtttgcaga tctttcaaga ctctagtcaa aatgagcaga 3000

acattttgca gttaatcata gagttgaaga tgaacgagaa gatgaacgag agctacttga 3060

ttgtgaacta gtaacgacca cttggagtcg cagtgtgtct gtaaaatgat gatgacagtg 3120

acatcataga ggacatagtg caagtgaaca gtttgatact gagcttggag cctcagtagt 3180

tctggataag tgagagctgt acgggaggtt gtaaccatga gctaccctgg ttggcagttg 3240

acatccgagg accagcactg gtcatcgcac agaaagaaaa ctttacacct tgagatccct 3300

ccattcttct taaacttcca agtgtatgtt tgatgggaga agaggggaag ggatcaacat 3360

ttaaaggctc tggaaggagt cagataactg tttttggctt caaatgtaga atgaatccct 3420

agattttcct ctactgagag aatgaaatct taacccattc cgttcccttg ttggtactca 3480

gaaacggata ccccaaagat gcattttgac atgctgaaag gccttagaag ctgccttaga 3540

agatctgtct aaccttgtct tgttggtgtg tggaagtggg aatgcccaca ggtgcacagg 3600

gagggagttt ctgaaattat ctgaccgaga aagcaaaaaa aaaaaatcaa ttgtcttaaa 3660

cccggtttct agggatctca ttaaagaact aggaaaaatt aaccacctgt taacagaaaa 3720

accaaactga aatgttttaa agagatttat tttgagccaa tatgagtgac catggcctgg 3780

ggaacacagg ctcaagaggt cctgagaaag tgtgcctgag acagtagaat tacagtttgg 3840

ttttgtacat tttaggtgga caggagttac agacaaagac ataaatcagt acatggaagg 3900

tacagattgt gttcagctgg gtgcagtggc tcacgcctgt aatcccagca ctttgggagg 3960

tcgaggtggg cgaatcgctt gaggtcagga gttcgagacc agcctggcta acatggtgaa 4020

accgtgtctc tacaaaaaat acaaaaaatt agccagggat ggtggcaggc acctgtagtc 4080

ctggagctac tcaggaggtt gaggcacgag aattgctgcg gaggttgtag tgagccgaga 4140

ccacaccact gcactccag 4159

<210> 3
<211> 21
<212> DNA

<213> Artificial Sequence

<220>
<223> primer

<400> 3
aggagaaaat tttgtgggat a 21

<210> 4
<211> 25
<212> DNA
<213> Artificial Sequence

<220>
<223> primer

<400> 4
caaaatcrat aacttaccta caaac 25

<210> 5
<211> 20
<212> DNA
<213> Artificial Sequence

<220>
<223> primer

<400> 5
tagattattg ggtaaaagaa 20

<210> 6
<211> 20
<212> DNA
<213> Artificial Sequence

<220>
<223> primer

<400> 6
aaaaccaaac taaactaaac 20

<210> 7
<211> 10
<212> DNA
<213> Artificial Sequence

<220>
<223> Tag sequence

<400> 7
aggaagagag 10

<210> 8
<211> 31
<212> DNA
<213> Artificial Sequence

<220>

<223> T7 promoter sequence

<400> 8
cagtaatacg actcactata gggagaaggc t 31

<210> 9
<211> 25
<212> DNA
<213> Artificial Sequence

<220>
<223> primer

<400> 9
taattgtttt gtttttttcg agttc 25

<210> 10
<211> 25
<212> DNA
<213> Artificial Sequence

<220>
<223> primer

<400> 10
aatcgataac ttacctacaa acgct 25

<210> 11
<211> 24
<212> DNA
<213> Artificial Sequence

<220>
<223> primer

<400> 11
atttatattt cgagattttt gtcg 24

<210> 12
<211> 22
<212> DNA
<213> Artificial Sequence

<220>
<223> primer

<400> 12
aaccaaacta aactaaacgc cg 22

<210> 13
<211> 176
<212> DNA
<213> Homo sapiens

<400> 13

aggagaaaac cctgtgggac aggggaggag ggtgagggct cctcttagga agttatttaa 60

gagccaactg tcttgtcttt cccgagtccg tttgaggaag tccccgaggc gcacagagca 120

agcccacgcg agggcacctc tggaggggag cgcctgcagg taagccaccg accctg 176

<210> 14
<211> 159
<212> DNA
<213> Homo sapiens

<400> 14

tagatcactg ggtaaaagaa ggggagcggc agcacgtatg gggtaggcgc tctcactact 60

cacatctcga gacctttgcc ggcgtagggc tgtccggggg gaacgacccg ccttttccgg 120

tatcggttgt catggcggcg cccagcccag cctggtttt 159

<210> 15
<211> 33
<212> DNA
<213> Artificial Sequence

<220>
<223> primer

<400> 15
gggatattaa gtggagttat tttggtttta gtt 33

<210> 16
<211> 21
<212> DNA
<213> Artificial Sequence

<220>
<223> primer

<400> 16
ccctcccaac atccttccta a 21

<210> 17
<211> 109
<212> DNA
<213> Homo sapiens

<400> 17

caactgtctt gtctttcccg agtccgtttg aggaagtccc cgaggcgcac agagcaagcc 60

cacgcgaggg cacctctgga ggggagcgcc tgcaggtaag ccaccgacc 109

<210> 18
<211> 100
<212> DNA

<213> Homo sapiens

<400> 18

```
actcacatct cgagaccttt gccggcgtag ggctgtccgg ggggaacgac ccgccttttc    60
cggtatcggt tgtcatggcg gcgcccagcc cagcctggtt                         100
```